(19) Europäisches Patentamt
European Patent Office
Office européen des brevets

(11) **EP 3 822 366 A1**

(12) **EUROPEAN PATENT APPLICATION**

(43) Date of publication:
**19.05.2021 Bulletin 2021/20**

(51) Int Cl.:
***C12Q 1/6881*** (2018.01)

(21) Application number: **19208964.7**

(22) Date of filing: **13.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ichnos Sciences SA**
**2300 La Chaux-de-Fonds (CH)**

(72) Inventors:
- **Christel, AEBISCHER-GUMY**
  **2300 La Chaux-de-Fonds (CH)**
- **Pierre, MORETTI**
  **2300 La Chaux-de-Fonds (CH)**
- **Martin, BERTSCHINGER**
  **2300 La Chaux-de-Fonds (CH)**

(54) **METHODS TO DETERMINE THE MONOCLONALITY OF A CELL POPULATION**

(57) The present invention relates to methods for determining the monoclonality of a cell population by the identification of genetic signatures, the determination of the presence of these genetic signatures in cells of this cell population and the application of statistical analysis to determine the probability that the cell population is monoclonal.

**FIG. 1**

SEQUENCE LISTING

<110> Glenmark Pharmaceuticals S.A

<120> Methods to determine the monoclonality of a cell population

<130> GBT3002-01-EP000

<160> 54

<170> PatentIn version 3.5

<210> 1
<211> 19
<212> DNA
<213> Artificial Sequence

<220>
<223> mAb LC A

<400> 1
tgaccaagag cttcaaccg                                                        19


<210> 2
<211> 18
<212> DNA
<213> Artificial Sequence

<220>
<223> mAb LC B

<400> 2
tcgcataaat ccacgggc                                                        18


<210> 3
<211> 18
<212> DNA
<213> Artificial Sequence

<220>
<223> mAb HC A

<400> 3
gacactgacc ctgacctg                                                        18


<210> 4
<211> 20
<212> DNA
<213> Artificial Sequence

<220>
<223> mAb HC B

<400> 4
ctctttcagt gtgacctgag                                                      20


<210> 5
<211> 19
<212> DNA

<213>  Artificial Sequence

<220>
<223>  mAb puro A

<400>  5
gaacctgccc ttctatgag                                                      19


<210>  6
<211>  19
<212>  DNA
<213>  Artificial Sequence

<220>
<223>  mAb puro B

<400>  6
gggcttgtac tctgtcatg                                                      19


<210>  7
<211>  20
<212>  DNA
<213>  Artificial Sequence

<220>
<223>  mAb neo A

<400>  7
ctgagaaggt gtccatcatg                                                     20


<210>  8
<211>  20
<212>  DNA
<213>  Artificial Sequence

<220>
<223>  mAb neo B

<400>  8
cagtcatagc caaacagtct                                                     20


<210>  9
<211>  20
<212>  DNA
<213>  Artificial Sequence

<220>
<223>  Universal A

<400>  9
gggtcaaatg cacaaatcat                                                     20


<210>  10
<211>  20
<212>  DNA
<213>  Artificial Sequence

<220>

<223> Universal B

<400> 10
ctcccaggtt caagtgatta                                                      20


<210> 11
<211> 18
<212> DNA
<213> Artificial Sequence

<220>
<223> BEAT LC A

<400> 11
cgtgaccaag agcttcaa                                                        18


<210> 12
<211> 19
<212> DNA
<213> Artificial Sequence

<220>
<223> BEAT LC B

<400> 12
ctctgggtca gctggatat                                                       19


<210> 13
<211> 20
<212> DNA
<213> Artificial Sequence

<220>
<223> BEAT HC A

<400> 13
ccgagaacaa ctactacacc                                                      20


<210> 14
<211> 18
<212> DNA
<213> Artificial Sequence

<220>
<223> BEAT HC B

<400> 14
attccaccag ctgcactt                                                        18


<210> 15
<211> 18
<212> DNA
<213> Artificial Sequence

<220>
<223> BEAT scFv-Fc A

<400> 15

gaatccgatg gcagcttc                                        18


<210>   16
<211>   20
<212>   DNA
<213>   Artificial Sequence

<220>
<223>   BEAT scFv-Fc B

<400>   16
cagttgaact tgacctcagg                                      20


<210>   17
<211>   19
<212>   DNA
<213>   Artificial Sequence

<220>
<223>   BEAT neo A

<400>   17
ggatgaggaa gcaccaggg                                       19


<210>   18
<211>   20
<212>   DNA
<213>   Artificial Sequence

<220>
<223>   BEAT neo B

<400>   18
atgatcaaat ggacaggtgg                                      20


<210>   19
<211>   18
<212>   DNA
<213>   Artificial Sequence

<220>
<223>   BEAT puro A

<400>   19
aacctgccct tctacgag                                        18


<210>   20
<211>   18
<212>   DNA
<213>   Artificial Sequence

<220>
<223>   BEAT puro B

<400>   20
ggtaggcttg tactcggt                                        18

```
<210>   21
<211>   19
<212>   DNA
<213>   Artificial Sequence

<220>
<223>   Set 1 A

<400>   21
tgaccaagag cttcaaccg                                                    19


<210>   22
<211>   18
<212>   DNA
<213>   Artificial Sequence

<220>
<223>   Set 1 B

<400>   22
tcgcataaat ccacgggc                                                     18


<210>   23
<211>   20
<212>   DNA
<213>   Artificial Sequence

<220>
<223>   Set 2 A

<400>   23
gcattctagt tgtggtttgt                                                   20


<210>   24
<211>   19
<212>   DNA
<213>   Artificial Sequence

<220>
<223>   Set 2 B

<400>   24
agaaaatctg gcagggatg                                                    19


<210>   25
<211>   20
<212>   DNA
<213>   Artificial Sequence

<220>
<223>   Integration site A 5' end 1

<400>   25
gcggggagaa gacagtaatg                                                   20


<210>   26
<211>   20
<212>   DNA
```

```
<213>  Artificial Sequence

<220>
<223>  Integration site A 5' end 2

<400>  26
gggcaatagg tctaagtgtg                                            20


<210>  27
<211>  15
<212>  DNA
<213>  Artificial Sequence

<220>
<223>  Integration site A 3' end 1

<400>  27
aaaattgccc ggggg                                                 15


<210>  28
<211>  26
<212>  DNA
<213>  Artificial Sequence

<220>
<223>  Integration site A 3' end 2

<400>  28
ggccgctgga aacacaagag attgtc                                     26


<210>  29
<211>  27
<212>  DNA
<213>  Artificial Sequence

<220>
<223>  Integration B 5' end 1

<400>  29
atattgtgca gccccaccct ctgcctc                                    27


<210>  30
<211>  28
<212>  DNA
<213>  Artificial Sequence

<220>
<223>  Integration B 5' end 2

<400>  30
ccggcgagaa atatgccctc acttgatc                                   28


<210>  31
<211>  18
<212>  DNA
<213>  Artificial Sequence

<220>
```

<223>    Integration B 3' end 1

<400>    31
ccatggtgga ctagcacc                                                          18


<210>    32
<211>    20
<212>    DNA
<213>    Artificial Sequence

<220>
<223>    Integration B 3' end 2

<400>    32
gttcttgccg gtcaagggtg                                                        20


<210>    33
<211>    22
<212>    DNA
<213>    Artificial Sequence

<220>
<223>    Junction 1 1

<400>    33
gtggccaact ccagctgtgg tg                                                     22


<210>    34
<211>    23
<212>    DNA
<213>    Artificial Sequence

<220>
<223>    Junction 1 2

<400>    34
cgaactccct acctcaggtt atc                                                    23


<210>    35
<211>    22
<212>    DNA
<213>    Artificial Sequence

<220>
<223>    Junction 2 1

<400>    35
gtggccaact ccagctgtgg tg                                                     22


<210>    36
<211>    28
<212>    DNA
<213>    Artificial Sequence

<220>
<223>    Junction 2 2

<400>    36

cgtgagtttt cgttccactg agcgtcag                                              28


<210> 37
<211> 22
<212> DNA
<213> Artificial Sequence

<220>
<223> Junction A 1

<400> 37
catggtgggc tagcaccggt cg                                                    22


<210> 38
<211> 23
<212> DNA
<213> Artificial Sequence

<220>
<223> Junction A 2

<400> 38
gacggacggc actcctcctc ctg                                                   23


<210> 39
<211> 24
<212> DNA
<213> Artificial Sequence

<220>
<223> WT_FW1

<400> 39
gctgaagcac aagaaataat gaca                                                  24


<210> 40
<211> 24
<212> DNA
<213> Artificial Sequence

<220>
<223> WT_REV1

<400> 40
agggaaccat gtatttcctt ttga                                                  24


<210> 41
<211> 20
<212> DNA
<213> Artificial Sequence

<220>
<223> Tg_FW1

<400> 41
ggaggcaggt ctctatctca                                                       20

```
<210>  42
<211>  24
<212>  DNA
<213>  Artificial Sequence

<220>
<223>  Tg_REV1

<400>  42
gctgaagcac aagaaataat gaca                                    24


<210>  43
<211>  20
<212>  DNA
<213>  Artificial Sequence

<220>
<223>  WT_2_FW1

<400>  43
gaaagaatgg aggtggctac                                        20


<210>  44
<211>  21
<212>  DNA
<213>  Artificial Sequence

<220>
<223>  WT_2_REV1

<400>  44
cccattaccc atgtatgctt a                                      21


<210>  45
<211>  24
<212>  DNA
<213>  Artificial Sequence

<220>
<223>  WT2_PROBE1

<400>  45
tcatcctgag gatctgctgt ggcc                                   24


<210>  46
<211>  18
<212>  DNA
<213>  Artificial Sequence

<220>
<223>  junction_1_FW1

<400>  46
ttaacaggtg gcgttcac                                          18


<210>  47
<211>  16
<212>  DNA
```

<213> Artificial Sequence

<220>
<223> junction_1_REV1

<400> 47
tggtcaggct ggtctc                                                                16


<210> 48
<211> 17
<212> DNA
<213> Artificial Sequence

<220>
<223> junction_1_PROBE1

<400> 48
tgggcccccg aaaggcc                                                               17


<210> 49
<211> 18
<212> DNA
<213> Artificial Sequence

<220>
<223> junction_2_FW1

<400> 49
ctccatgtac tcgtgcag                                                              18


<210> 50
<211> 18
<212> DNA
<213> Artificial Sequence

<220>
<223> junction_2_REV1

<400> 50
tccactgagc gtcagata                                                              18


<210> 51
<211> 26
<212> DNA
<213> Artificial Sequence

<220>
<223> junction_2_PROBE1

<400> 51
cgaagatcag cagttcctct gttgcc                                                     26


<210> 52
<211> 20
<212> DNA
<213> Artificial Sequence

<220>

<223>    junction_A_FW1

<400>    52
ggatgtcatt gtcactcaag                                                    20


<210>    53
<211>    18
<212>    DNA
<213>    Artificial Sequence

<220>
<223>    junction_A_REV1

<400>    53
cagcccatga gaagtacc                                                      18


<210>    54
<211>    21
<212>    DNA
<213>    Artificial Sequence

<220>
<223>    junction_A_PROBE1

<400>    54
ccagatctca atgggtttgg a                                                  21


**Description**

**TECHNICAL FIELD**

The present invention relates to methods for determining the monoclonality of a cell population by the identification of genetic signatures, the determination of the presence of these genetic signatures in cells of this cell population and the application of statistical analysis to determine the probability that the cell population is monoclonal.

**BACKGROUND**

A monoclonal cell population is a population of cells all descending from a single parental progenitor. The use of monoclonal cell populations is often required for the industrial production of polypeptides and proteins such as enzymes, hormones, growth factors and antibodies in order to assure structural and functional homogeneity of the product. For example, for the manufacturing of biological therapeutics, e.g. therapeutic proteins such as therapeutic antibodies, the use of monoclonal cell lines is required to ensure consistent quality throughout the life cycle of a product (Frye et al., 2016; Walsh, 2014; Zhu, 2012). To achieve this, one key point requested by regulatory agencies is to demonstrate the monoclonality of the cell banks, i.e. providing evidence that the entire cell population is derived from a single cell progenitor (ICH Q5D).

The monoclonality of a cell bank is generally ensured by the process used for its generation. Different statistical approaches have been proposed (Coller & Coller, Hybridoma, 2(1), 91-96,1983; Evans et al., Biotechnol Prog, 31(5), 1172-1178, 2015; Lefkovits & Waldmann, Immunol Today, 5(9), 265-268, 1984; Quiroz & Tsao, Biotechnol Prog, 32(4), 1061-1068, 2016; Waldmann & Lefkovits, Immunol Today, 5(10), 295-298, 1984). However the statistics are influenced by many factors (Staszewski, Statistics in medicine, 9, 457-461,1990), including cell clumping (Klottrup, Miro-Quesada, Flack, Pereda, & Hawley-Nelson, 2017) or differences in survival rates (Zhou et al., Biotechnol Prog, 2017) that need to be carefully assessed. The FDA (US Food and Drug Administration) considers two rounds of limiting dilution with a seeding density lower than 1 cell per well an acceptable procedure to generate a monoclonal cell line (Kennett, 2014). In case the process used for the generation of the cell bank is not fully compliant with regulatory guidance and no additional data are available (e.g. imaging data), it used to be common practice to change the cell line to ensure compliance. This approach, however, demands a considerable investment in time and cost and requires intensive communication with the regulatory agencies who may be concerned about consistency of product quality. In industry there is therefore a growing interest and need of methods to determine the monoclonality of established cell lines.

**SUMMARY OF THE INVENTION**

The present invention relates to methods for determining the monoclonality of a cell population. Monoclonal cell populations are often used in the industrial production of polypeptides and proteins to assure homogeneity of the product. For instance, in the production process of biological therapeutics, monoclonality of production cell lines is required to generate a robust process ensuring product consistency and safety (Walsh, Nature Biotechnology, 32(10), 992-1000,2014; Zhu,Biotechnol Adv, 30(5), 1158-1170, 2012). In fact, monoclonal cell lines, which are expected to be homogeneous, respond in a reproducible manner to minor process changes. In contrast, subpopulations within a polyclonal cell line may react differently and this may lead to measureable and unexpected differences in product quality attributes. Monoclonality can be ensured by the cloning procedure (e.g. by two rounds of limiting dilution with a seeding density ≤1 cell/well). If the cloning procedure is not sufficient to ensure the monoclonality of the cell line, a demonstration of the homogeneity of the cell line is required.

The present invention discloses methods to demonstrate the monoclonality of a cell population by identifying genetic signatures that link the cells forming said population to a single same progenitor. The present invention also relates to methods to demonstrate the monoclonality of a cell population generated from a host cell which has undergone transfection of nucleic acid material encoding the polypeptide of interest. In this case, the genetic signatures may result from the integration of the transfected nucleic acid material in the host cell genome. The disclosed methods are independent to the process of generation of the cell population. Given that assuring monoclonality is crucial to the manufacturing of protein products, such as therapeutic biologics, the present invention represents a valuable response to the necessity and the interest of the industry, especially when demonstration of compliance with regulatory agencies requests is needed.

In particular the present invention discloses a method for determining the monoclonality of a cell population comprising a plurality of cells that express a polypeptide of interest, wherein said method is characterized by the steps of:

(a) identifying at least one cell of the cell population having at least one genetic signature;
(b) generating at least one clone(s) of a single cell from said cell population by applying at least one single cell cloning step;

(c) detecting which of said at least one clone(s) comprises said at least one genetic signature;

(d) determine the absence of any of said at least one clone which do not comprise said at least one genetic signature;

(e) confirming the monoclonality of said cell population by applying statistical analysis to determine within a predetermined confidence interval that the probability that said cell population is not monoclonal is less than a predetermined value, wherein said predetermined value is calculated using the formula

$$\frac{p + \frac{z^2}{2(Clones)}}{1 + \frac{z^2}{(Clones)}} + \frac{z}{1 + \frac{z^2}{(Clones)}} \sqrt[2]{\frac{p(1-p)}{(Clones)} + \frac{z^2}{4(Clones)^2}}$$

or the formula

$$One\ sided\ 95\%\ Not\ monoclonal = \mathrm{Exp}\left((-0.0058) - 0.5029 \times ln(Clones) - 0.0824 \times ln(Clones)^2 + 0.0045 \times ln(Clones)^3\right)$$

In one aspect of the present invention, the genetic signature is associated to a locus comprising the coding sequence of the polypeptide of interest. According to a different aspect of the present invention, the genetic signature is not associated to a locus comprising the coding sequence of said polypeptide of interest.

According to one aspect of the present invention, the predetermined value is equal to or greater than 0.05% and equal to or less than 2%.

According to another aspect of the present invention, the predetermined confidence interval is equal to or greater than 90% and equal to or less than 98%.

In one aspect of the present disclosure, the number clones analyzed is between 5 and 10000.

In one aspect of the present invention, the polypeptide of interest is encoded by a coding sequence naturally present into the genome of said cell population. Accordingly, the genetic signature is selected from the group comprising deletions, insertions, duplications and substitutions.

In another aspect of the present invention, the cell population is produced starting from a host cell which has undergone transfection of nucleic acid material comprising the coding sequence of said polypeptide of interest, and wherein said nucleic acid material integrates in the genome of said host cell.

In one embodiment, the genetic signature results from the integration of the transfected nucleic acid material into the host cell genome and said genetic signature is selected from the group comprising the integration sites, concatemer junctions and mutations, wherein said integration site include but are not limited to the sites of insertion of the genetic material in the host cell genome; said concatemer junctions include but are not limited to the fused ends of two or more of transfected nucleic acid material; and wherein said mutations include but is not limited to deletion, insertions, duplications and substitutions.

According to an aspect of the present invention, the cell population and/or host cell is selected from the group comprising insect, plant and mammalian cells. According to a preferred aspect of the present invention, the host cell is a mammalian cell. More preferably the host cells are CHO cells.

In one embodiment of the present invention, the polypeptide of interest is a protein; preferably an antibody or an antibody fragment.

The present invention also relates to a cell population subjected to the disclosed method.

The term "cell population" or "existing cell population" as used in the present invention, indicates a group of cells; in particular a group of cells that express one or more polypeptides of interest.

A cell population may derive from a single clone, namely a group of cells descending from the same single cell, or from more than one clone. Based on the origin, said cell population may be monoclonal or polyclonal. In the present invention, the term "monoclonal" refers to a cell population which is exclusively composed by descendants of a defined single parental progenitor. The term "monoclonality" therefore indicates the characteristic of a cell population of descending from a single parental progenitor. The term "polyclonal" refers to a cell population which is composed by descendants of more than one parental progenitor.

According to the present invention the cell population is a population of cells where no exchange of genetic material with the outside environment occurs unless the cells undergo transfection. The cell population may be a population of higher eukaryote cells comprising insect cells, plant cells and mammalian cells. The cell population may be a cell line derived multicellular organisms. Examples of invertebrate cells include insect cells such as Spodoptera frugiperda (caterpillar), Aedes augypti (mosquito), Aedes albopictus (mosquito), Drosophila melanogaster (fruitfly) and Bombyx mori., Examples of plant cell cultures include cotton, corn, potato, soybean, petunia, tomato, and tobacco. Cell populations for expressing the antibodies are preferably mammalian cells which include Chinese hamster ovary (CHO) cells, NSO mouse myeloma cells, human cervical carcinoma (HeLa) cells, COS cells, SP2 cells and human embryonic kidney (HEK) cells.

In the present invention, the terms "protein" and "polypeptide" are used interchangeably to include a series of amino acid residues connected to the other by peptide bonds between the alpha-amino and carboxy groups of adjacent residues. The term "polypeptide of interest" is used herein to indicate a single amino acid chain constituting the polypeptide as well as proteins consisting of more than one amino acid chain. Non limiting examples of polypeptides of interest produced by a cell population comprise enzymes, hormones, regulatory proteins, antigens, antibodies. The polypeptide of interest expressed by the cell population, may be encoded by a coding sequence naturally present in the cells genome. Alternately, the polypeptide of interest may be expressed by a cell population generated starting from a host cell which has undergone transfection of nucleic acid material comprising the coding sequence of said polypeptide of interest. Additionally, the polypeptide of interest may be a naturally occurring polypeptide or the product of genetic engineering, e.g. the polypeptide of interest may be produced by recombinant DNA techniques or artificially synthetized.

In a preferred embodiment of the present invention the polypeptide of interest is a therapeutic protein; more preferably the polypeptide of interest is a therapeutic antibody.

The term "antibody" as referred to herein includes whole antibodies and any antigen binding fragments or single chains thereof. An "antibody" refers to a glycoprotein comprising at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR) which are hypervariable in sequence and/or involved in antigen recognition and/or usually form structurally defined loops, interspersed with regions that are more conserved, termed framework regions (FR or FW). Each VH and VL is composed of three CDRs and four FWs, arranged from amino- terminus to carboxy- terminus in the following order: FW1, CDR1, FW2, CDR2, FW3, CDR3, FW4. The term "full length antibody" as used herein includes the structure that constitutes the natural biological form of an antibody, including variable and constant regions. For example, in most mammals, including humans and mice, the full length antibody of the IgG class is a tetramer and consists of two identical pairs of two immunoglobulin chains, each pair having one light and one heavy chain, each light chain comprising immunoglobulin domains VL and CL, and each heavy chain comprising immunoglobulin domains VH, CHI (Cyl), CH2 (Cy2), and CH3 (Cy3). In some mammals, for example in camels and llamas, IgG antibodies may consist of only two heavy chains, each heavy chain comprising a variable domain attached to the Fc region. Antibody fragments include, but are not limited to, (i) the Fab fragment consisting of VL, VH, CL and CHI domains, including Fab' and Fab'-SH, (ii) the Fd fragment consisting of the VH and CHI domains, (iii) the Fv fragment consisting of the VL and VH domains of a single antibody; (iv) the dAb fragment (Ward ES et al, (1989) Nature, 341 : 544-546) which consists of a single variable, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al, (1988) Science 242: 423-426; Huston JS et al, (1988) Proc. Natl. Acad. Sci. USA, 85: 5879-83), (vii) bispecific single chain Fv dimers (PCT/US92/09965), (viii) "diabodies" or "triabodies", multivalent or multispecific fragments constructed by gene fusion (Tomlinson I & Hollinger P (2000) Methods Enzymol. 326: 461-79; WO94/13804; Holliger P et al, (1993) Proc. Natl. Acad. Sci. USA, 90: 6444-48) and (ix) scFv genetically fused to the same or a different antibody (Coloma MJ & Morrison SL (1997) Nature Biotechnology, 15(2): 159-163).

The term "host cells" as used herein include any cells which are capable of growing in culture and expressing the polypeptide of interest. The host cell line may belong to the cell population types specified above. In particular, cell lines suitable for the expression of the antibody include and are not limited to mammalian, insect, plant cells. Cell lines often used for the expression and production of therapeutic antibodies are mammalian cells lines such as Chinese hamster ovary (CHO) cells, NSO mouse myeloma cells, human cervical carcinoma (HeLa) cells and human embryonic kidney (HEK) cells.

The term "cell transfection" refers to the introduction of exogenous nucleic acid material into a host cell. When the polypeptide codified by the artificially introduced nucleic acid is expressed by the cells, it provides the genetically modified cells with properties different than the respective wild type form. The introduced nucleic acid can be DNA or RNA. Examples of techniques commonly used for introducing exogenous nucleic acid into the host cells include chemical-based methods, where the transfection is mediated by transfection reagents such as calcium-phosphate, liposomes, cationic polymers or dendrimers; physical-based method such as electroporation and microinjection; and virus-based methods where virus infection mediates gene delivery. Using these techniques, transient or stable transfection can be achieved. In the transient transfection the nucleic acid sequence does not integrate into the genome of the host cell, therefore the expression of the protein codified by the exogenous genetic material is limited in time, while stable transfection is achieved when the cells integrate the foreign genetic material in their genome, giving rise to a stable transfected cell line.

The transfected nucleic acid material, referred inhere also as "transgene", comprises at least a polynucleotide sequence encoding a polypeptide of interest, here referred as "coding sequence". The nucleic acid material that encode the polypeptide of interest of the present invention may be incorporated into a vector, preferably an expression vector in order to express the polypeptide of interest. The term "expression vector" as used herein includes an isolated and purified DNA molecule which upon transfection into an appropriate host cell provides for a high-level expression of a recombinant gene product within the host cell. In addition to the DNA sequence coding for the recombinant or gene product the expression vector comprises regulatory DNA sequences that are required for an efficient transcription of the DNA coding sequence into mRNA and for an efficient translation of the mRNAs into proteins in the host cell line. A variety of expression vectors may be utilized for protein expression. Expression vectors may comprise self-replicating extrachromosomal vectors or vectors which integrate into a host genome. Expression vectors are constructed to be compatible with the host cell type. Non limiting examples of expressing vectors include plasmid vectors, viral vectors and cosmids.

The expression vector may contain a selectable marker, such as a gene for antibiotic resistance. Upon transfection of the expression vector, host cells may be subjected to selection pressure, for instance by introducing such antibiotic in the cell culturing medium. This selection step allows selecting cells where the vector has been successfully transfected.

Selected cells may be further subjected to at least one single cell cloning step. The term "single cell cloning" as used herein indicates the process of creating clones from single cells that have been separated one from the other one. The single cell cloning step may be carried out using different techniques that allow single cell isolation, non-limiting examples of such techniques include one or more rounds of limiting dilutions (i.e. seeding cells at density ≤ 1 cell/well), cell sorting or growing the cell on semi-solid media.

When host cells are transfected with exogenous nucleic acid material encoding for the polypeptide of interest, the nucleic acid material may integrate into the host cell genome. Upon transfection host cell may have been subjected to at least one selection step, and to at least one cloning step.

The term "genetic signature" as used herein refers to any genetic feature that can be specifically associated to a clone, therefore unique for said clone. A genetic signature may be the result of accumulation of random mutations in the cellular genome. The term "mutation" as used in the present invention, refers to any change in a polynucleotide sequence, e.g. in the DNA of a cell. Mutations include but are not limited to substitutions, wherein at least a nucleotide base is replaced by another one or wherein more than one a nucleotide bases are replaced; deletions, wherein at least a nucleotide base is removed; insertions, wherein at least a nucleotide base is added; duplications, wherein at least a nucleotide base is copied one or more times; repeat expansions, wherein the number of times in which normally a short DNA sequence is repeated, increases.

For host cells transfected with nucleic acid material, such as a vector comprising the nucleotide sequence encoding for the polypeptide of interest, when said nucleic acid material integrates into the genome of the host cell, genetic signatures may be genetic features resulting from the integration event. Genetic signatures resulting from the integration event include but are not limited to integration sites, concatemer junctions and mutations. As used herein the term "integration sites" refers to one or more sites of insertion of the genetic material in the host cell genome, e.g. plasmid-host cell DNA fusion sites. As used herein the term "concatemer junctions" refers to one or more junctions resulting from the assembly of concatemers; when more than one copy of the transfected genetic material, such as DNA, is present in the nucleus of the host cell, DNA repair mechanisms may lead the joining of said copies and the formation of concatemers, which is followed by the integration of the concatemers is single or multiple locations in the host genome. Examples of concatemers junctions include but are not limited to vector-vector junctions such as plasmid-plasmid junctions. The term "mutations" as specified above refers to any change in a polynucleotide sequence, such as in the transfected nucleic acid sequence.

In the present invention the genetic signatures associated to a locus which encodes the polypeptide of interest are identified by Target Locus Amplification (TLA) technology followed by Next Generation Sequencing (NGS). Alternatively, techniques known in the art such as inverted PCR followed by Sanger sequencing, whole genome sequencing approaches, FISH or Southern Blot could be used for genetic signature determination.

Following the identification of the genetic signatures, a single cell cloning step is applied to generate clones of the cell population. The term "single cell cloning step" as used inhere refers to the step of producing clones derived from single cells, such as the single cells forming the cell population. Various methods can be used for the single cell cloning step as specified above.

In the present invention, the clones generated upon the single cell cloning step are analyzed by qPCR for the presence of said genetic signatures. Alternative techniques to detect the clones wherein the genetic signatures are present include but are not limited to FISH, Southern Blot, whole genome sequencing approaches and TLA followed by NGS.

According to the present invention, following the detection of the clones presenting the identified genetic features, statistical analysis is applied to determine within a confidence interval the probability that the cell population from which the clones are generated is not monoclonal. In particular statistical analysis is applied to determine within a predetermined confidence interval the probability that said cell population is not monoclonal is less than a predetermined value. In a particular embodiment of the current disclosure, the binomial "contaminating population detected/no contaminating population detected" score method of confidence interval (Chapter 2.2 in (Wallis, Journal of Quantitative Linguistics, 20(3), 178-208, 2013)) is applied using SAS/JMP® software. In one embodiment the following formula is used to calculate the One Sided 95% Not Monoclonal:

(Formula 1)

$$\frac{p + \frac{z^2}{2(Clones)}}{1 + \frac{z^2}{(Clones)}} \pm \frac{z}{1 + \frac{z^2}{(Clones)}} \sqrt[2]{\frac{p(1-p)}{(Clones)} + \frac{z^2}{4(Clones)^2}}$$

Wherein $p=0$ indicates no contamination of the cell population and $z=1.645$, being the $z$ score for 95% confidence interval. To evaluate the population of cells that are potentially not monoclonal sample sizes from 10 to 8000 cells were evaluated, in particular for *Clones* values equal to 10, 20, 30, 60, 90, 180, 250, 500, 1000, 2000, 4000 and 8000 and the one-sided probability of monoclonal and not monoclonal are calculated using the formula derived from Wallis, 2013.

Next the following formula has been derived from the fitting of the One Sided 95% Not Monoclonal obtained from Formula 1 by the log of *Clones* (ln(*Clones*)).

(Formula 2)

$$One\ sided\ 95\%\ \text{Not monoclonal} = \text{Exp}\left((-0.0058) - 0.5029 \times ln(Clones) - 0.0824 \times ln(Clones)^2 + 0.0045 \times ln(Clones)^3\right)$$

This formula can be used to calculate the one-sided probability of not monoclonal for a given value of *Clones.*

The term "predetermined value" as used herein refers to the upper limit of the confidence interval that the probability said cell population is not monoclonal. In a certain embodiment said predetermined value approaches 0%. In another embodiment said predetermined value is equal to or greater than about 0.01% and equal to or less than about 5%; or equal to or greater than about 0.05% and equal to or less than about 2.5%; or equal to or greater than about 0.5% and equal to or less than about 2%; or is equal or less about 1.5%. More specifically the predetermined value is selected from the group comprising about 0.01%, about 0.05%, about 0.1%, about 0.5%, about 1%, about 1.5%, about 2%, about 2.5% and about 5%. The present invention also includes predetermined value at intervals of 0.01%, 0.1%, 0.2%, 0.3%, 0.4%, 0.5%, 0.6%, 0.7%, 0.8%, 0.9% and 1% between the above said values.

The term "predetermined confidence interval" as used herein refers to the probability that the upper limit of said confidence interval is equal to or less than the predetermined value.

In one embodiment said predetermined confidence interval is equal to or greater than about 80% and equal to or less than about 99,9%; or is equal to or greater than about 85% and equal to or less than about 99%; is equal to or greater than about 90% and equal to or less than about 96%; is about 95%. More specifically, predetermined confidence interval is selected from the group comprising about 80%, about 85%, about 90%, about 95% about 98%, about 99%. The present invention also includes predetermined confidence interval at intervals of 0.1%, 0.2%, 0.5%, 0.7%, 1%, 2%, 3%, 4% 5% 6% 7%, 8%, 9% between the above said values.

In one embodiment the number of clones analyzed to determine said confidence interval is equal to or greater than 5 and equal to or less than 10000; or equal to or greater than 10 and equal to or less than 8000. In another embodiment number of clones analyzed to determine said confidence interval is greater than 10000. In particular the number of clones analyzed to determine said confidence interval is selected from the group comprising 5, 10, 20, 30, 60, 90, 180, 250, 500, 1000, 2000, 4000, 8000, 10000, more than 10000. The present invention also includes clones analyzed to determine said confidence interval at intervals of 5, 10, 50, 100, 500, 1000 between the above said values.

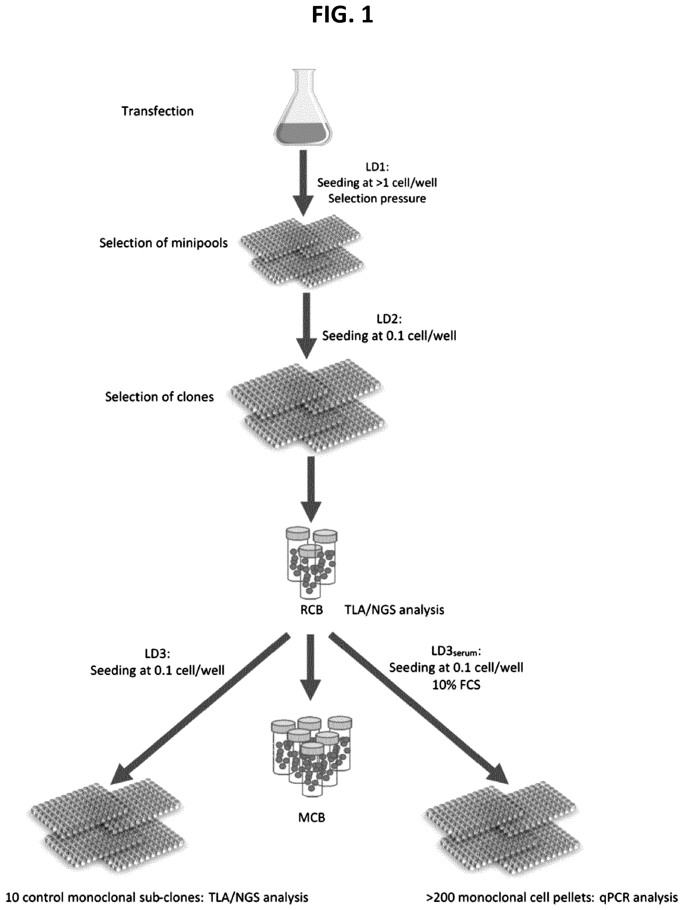

**Figure 1: Schematic overview of cell line development and monoclonality demonstration.**

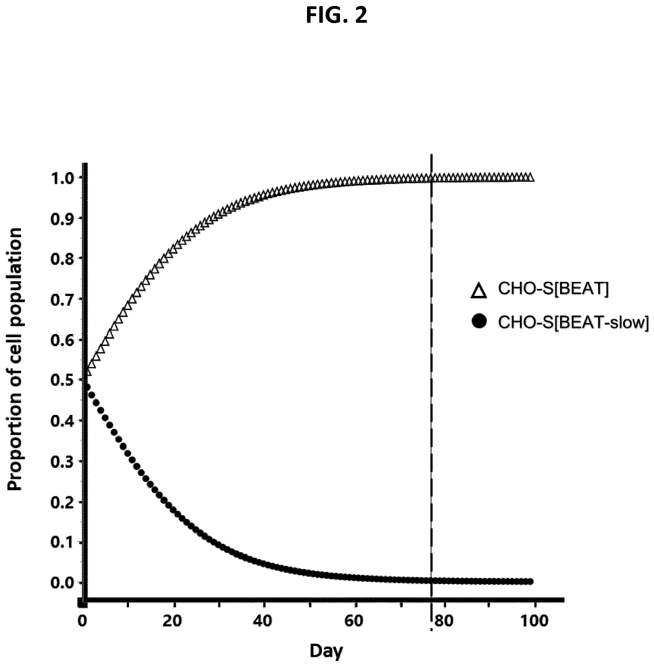

**Figure 2: Theoretical redistribution of cell populations.** Assuming clone CHO-S[mAb] was deposited in the same well as Clone CHO-S[BEAT-slow] during the limiting dilution step, a difference of 13% in growth rate would lead to rapid overgrow of CHO-S[BEAT] (open triangles) over CHO-S[BEAT-slow] (full circles). The dashed line represents the time-point where the RCB of the CHO-S[BEAT] was frozen after limiting dilution step.

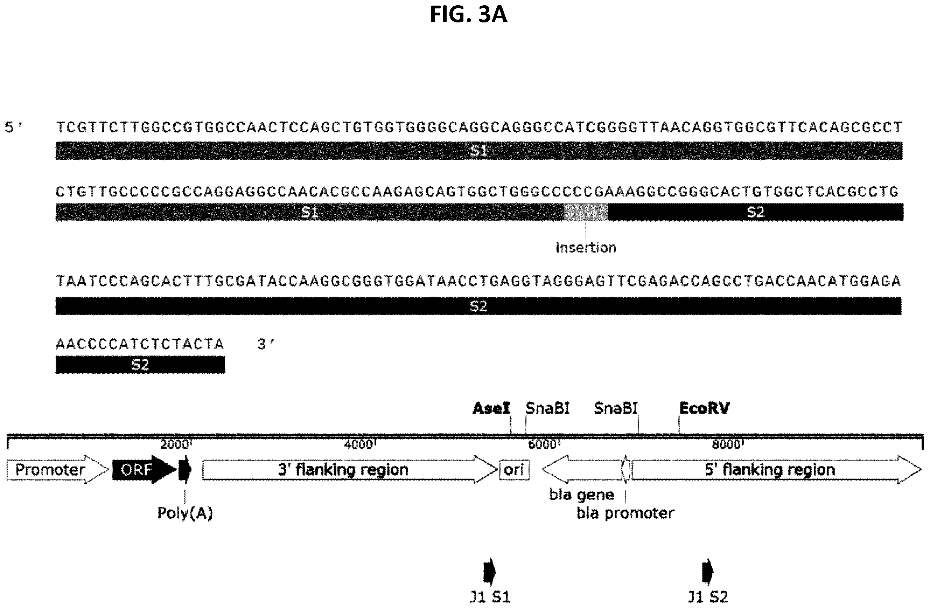

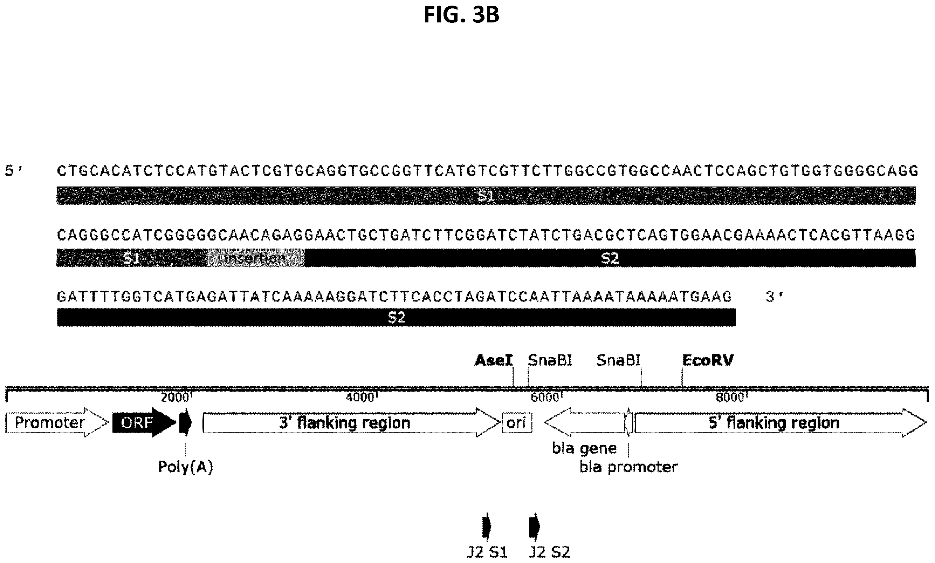

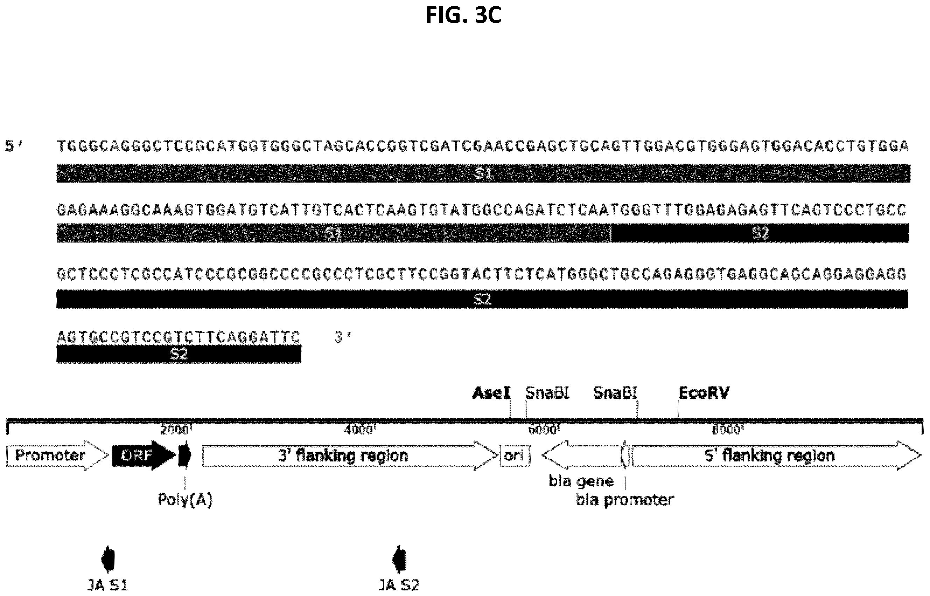

**Figure 3:** Three plasmid-plasmid fusions of special interest were detected in clone CHO-S[BEAT]. Junction 1 (A) and Junction 2 (B) contained non-plasmid DNA stretches between the two plasmid ends. Junction A (C) was the result of a complex rearrangement of the plasmid sequences before fusion. Sequences of the plasmid-plasmid fusions are depicted as well as the alignment of these sequences to the plasmid maps. The restriction sites depicted on the plasmid maps (AseI, SnaBI and EcoRV) correspond to the restriction enzymes used for linearization of the plasmid prior to transfection.

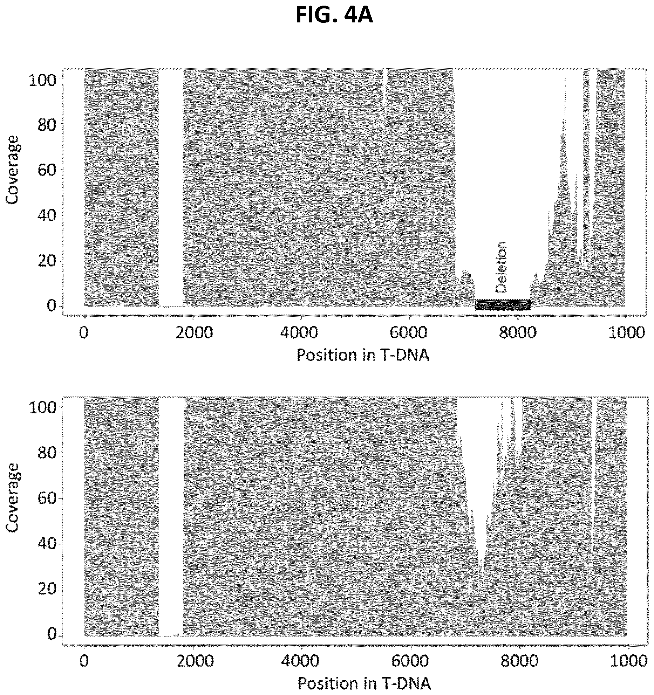

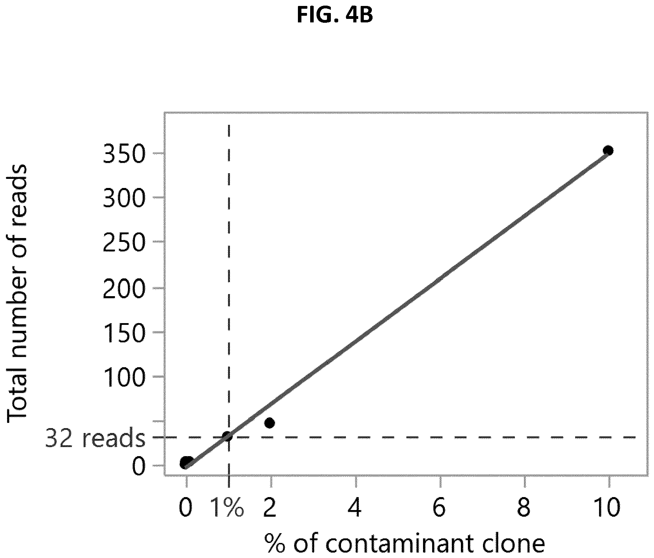

**Figure 4:** Assessment of cell population homogeneity by TLA/NGS using a specific deletion identified in clone CHO-S[mAb]. (A) TLA/NGS results for the CHO-S[mAb] RCB (top panel) and for the corresponding parental pool (bottom panel). (B) Mixture experiment with increasing amount of contaminating cell line not bearing the deletion. The coverage at three different points was determined and plotted as "total number of reads".

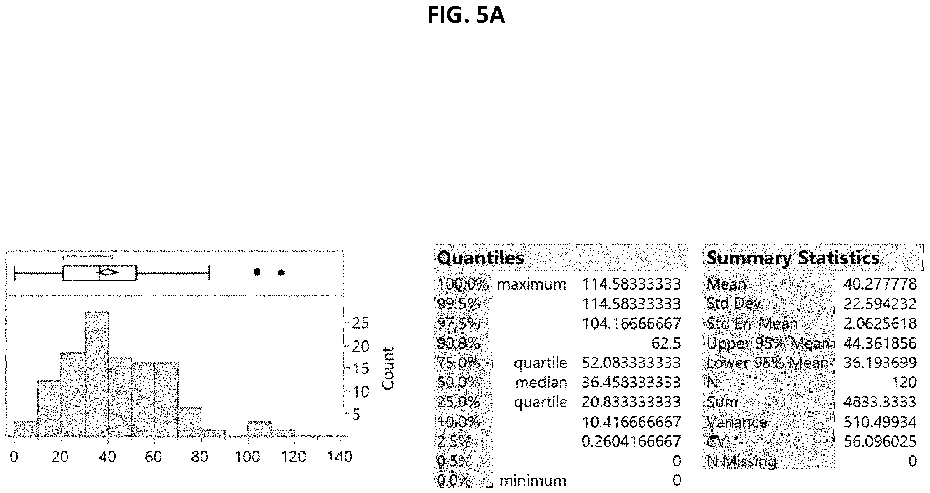

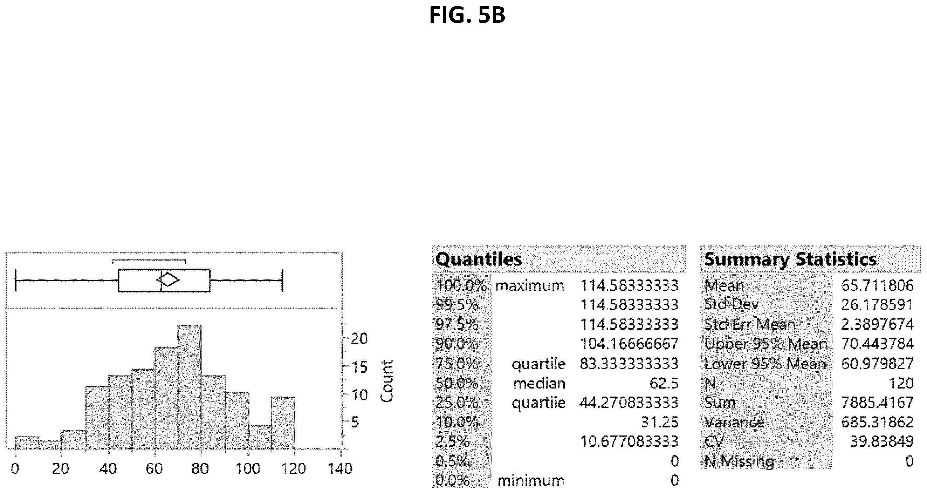

**Figure 5:** Growth rate in LD3$_{Serum}$: A total of 120 plates were analyzed for clone CHO-S[mAb] (A) and clone CHO-S[BEAT] (B), respectively. With a seeding density of 0.1 cell/ 96 well, an efficiency of 100% corresponds to 10 wells (9.6) showing growth.

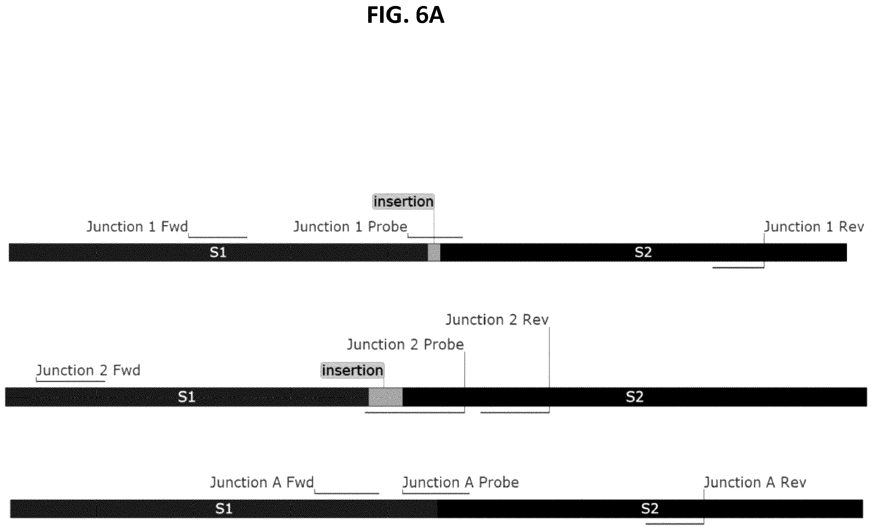

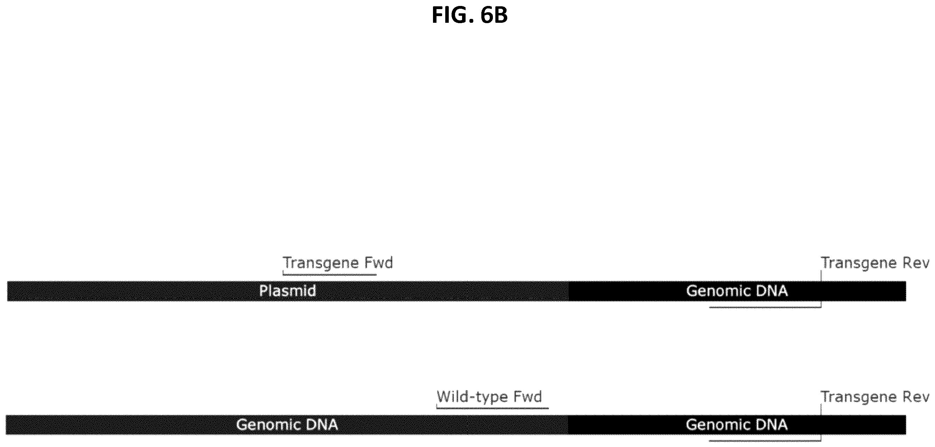

**Figure 6:** Design of the qPCR assays. (A) Plasmid-plasmid fusions: qPCR based on a hydrolysis probe spanning the junction between the two plasmid sequences, including the inserted nucleotides if present. (B) Genome plasmid fusion: qPCR based on SYBR green incorporation: the same reverse primer is used for both the Tg and the WT assay, resulting in an amplification for the Tg assay from the chromosome having integrated the transgene, and the an amplification for the WT assay from the intact chromosome(s).

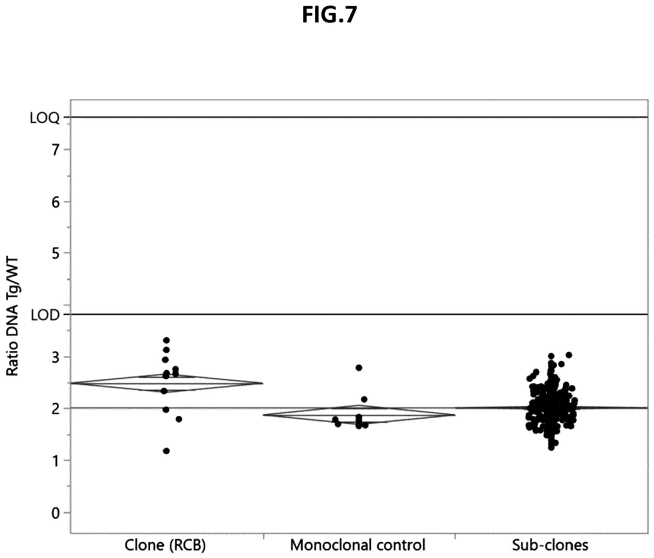

**Figure 7:** Normalized signals of qPCR performed on 219 sub-clones of CHO-S[mAb] (without P2B01 and P2H07 in which either Tg or WT signal was missing). For all samples, signal is below the LOD for a secondary subpopulation having lost the genetic feature.

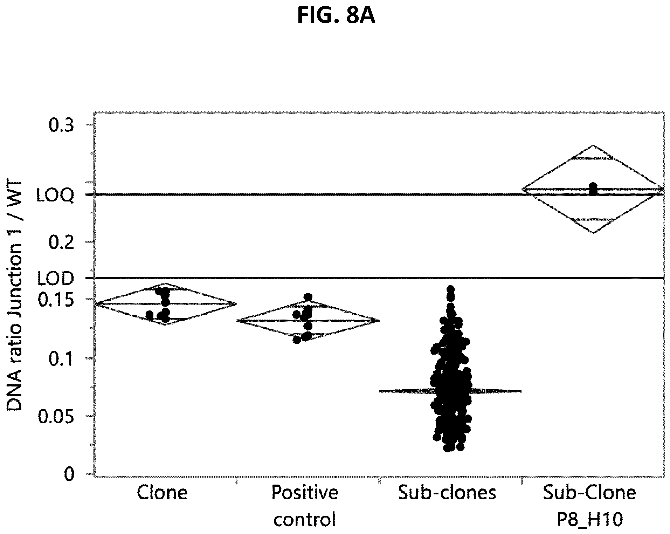

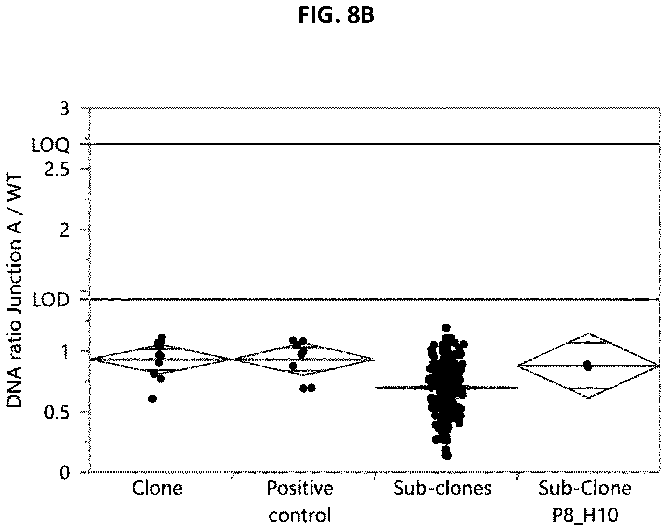

**Figure 8:** Normalized signals of qPCR performed on 207 sub-clones of CHO-S[BEAT] for (A) Junction 1 and (B) Junction A. Junction 2 assay did not allow quantification. For Junction A, all signals are below the LOD for contaminating subpopulation. For Junction 1, one sample (P8H10) is above the LOQ (duplicate value, the triplicate was an outlier) with 71% of contaminant sub-population having lost this specific plasmid-plasmid junction.

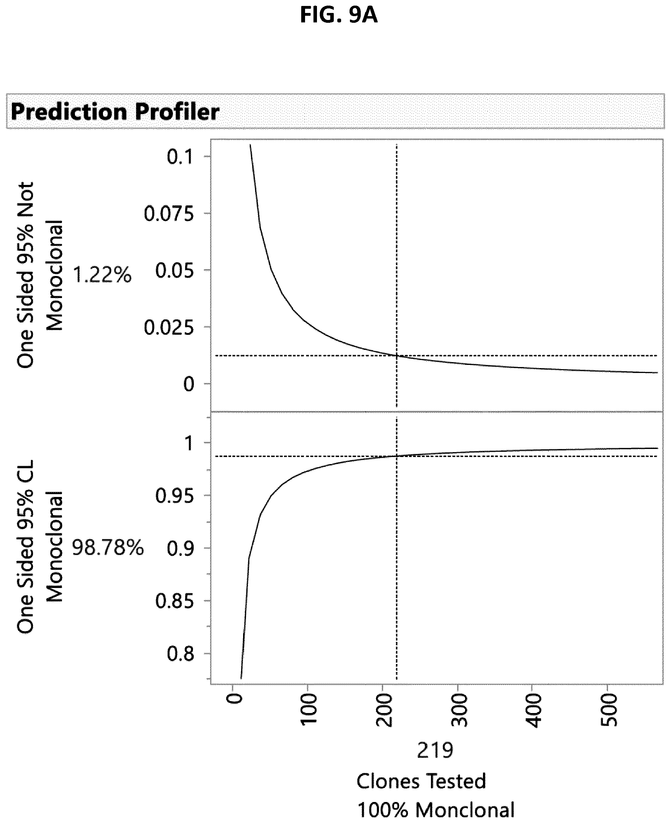

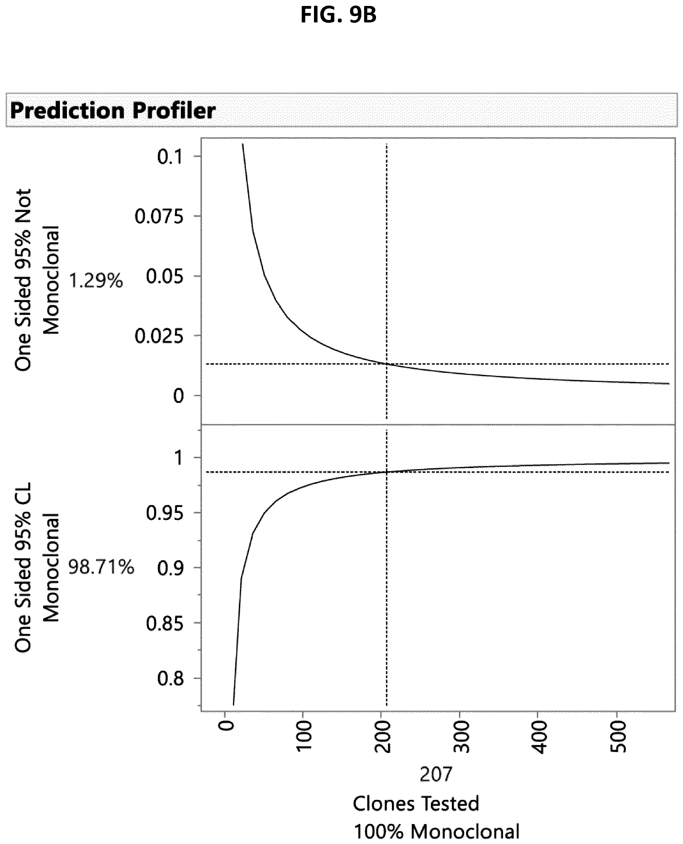

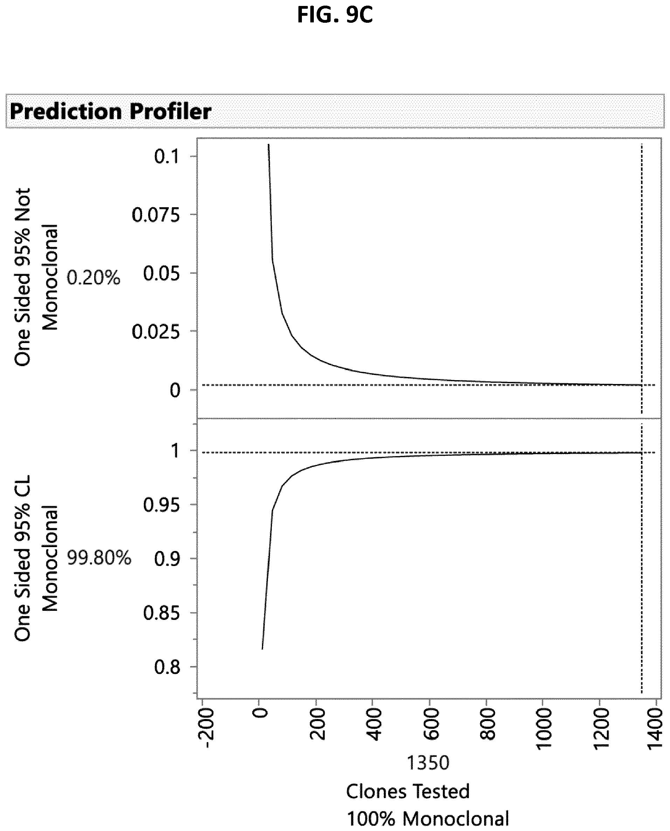

**Figure 9:** The graph shows the upper limit of the one sided 95% confidence interval for probability of not monoclonal and monoclonal based on the number of samples analyzed. For CHO-S[mAb] (A) and CHO-S[BEAT] (B) the upper limit of the 95% confidence interval is 1.22% and 1.29% respectively. Decreasing the upper limit of the 95% confidence interval below 0.2% would have required the analysis of more than 1350 sub-clones (C).

**EXAMPLES**

**Example 1: Introduction to the identification of genetic signatures**

The cellular genome is prone to accumulate random mutations over time and the accumulation of these acquired mutations makes each clone genetically unique. As soon as a specific genetic feature can be associated specifically to a clone (in contrast to the parental cell population), it can be considered to be an identifying characteristic of this clone (a "signature") and can be used to evaluate the homogeneity of the cell bank. For recombinant cell lines, the integration of the transgene in the host cell genome is a special case of mutation that occurred in the first ancestor of the cell line.

Following transfection, multiple plasmids are present in the nucleus of the cells and different DNA repair mechanisms may be involved in concatemerization of plasmids and integration in a single or in multiple random locations in the host genome. In order to avoid random plasmid linearization that would lead to disruption of the transgene expression cassette, as well as to maximize the likelihood of transgene integration, plasmids usually are linearized at specific sites outside the expression cassette prior to transfection. DNA repair mechanisms may introduce modifications of plasmid sequences during concatemerization, e.g. deletion of bases at the ends of the plasmids or insertion of stretches of non-plasmid related DNA nucleotides between two plasmid sequences. Some even more dramatic modifications of plasmid sequences can also occur, such as deletions of large portions of the plasmid sequence.

During the cell line development process, the cells usually are selected for high and stable expression of the transfected transgenes. Cells selected in this manner have the transgene DNA stably integrated in their genome. A stable clone is thus characterized by a specific transgenic DNA integration event, itself unique with regards to the precise integration site(s) of the plasmid DNA in the host genome (except in the case of targeted integration), variations in the assembly of the integrated concatemers (plasmid-plasmid fusions) or in sequence integrity (re-arrangements or mutations in plasmid backbones). In the present invention we propose to use these unique genetic features to identify clones and thus to determine the homogeneity of an existing cell population.

The methods disclosed by the present invention to determine the monoclonality of cell lines are independent of the process used for their generation, based on an analysis of the integrated transgenes. The methods includes the use of target locus amplification (TLA) followed by next generation sequencing (NGS), which allows a detailed analysis of the transgene locus and the identification of unique genetic features in a specific clone, and analysis of sub-clones generated from the cell banks, followed by statistical analysis, which allows the assessment of the homogeneity of the cell bank based on the presence or absence of the genetic features identified in the clone.

**Example 2: Materials and Methods**

Generation of the cell lines used in this study

The monoclonal status of two different cell lines was demonstrated in this study. The two independently generated cell lines will be referred to as CHO-S[mAb] and CHO-S[BEAT]. The cell line CHO-S[mAb] is derived from the CHO-S (Invitrogen, Carlsbad, CA) parental cell line. It expresses a monoclonal antibody of the IgG1κ format and was generated by the co-transfection of four different plasmids expressing the light chain, heavy chain and two resistance genes for antibiotics used as selection agents, respectively. CHO-S[BEAT] is also derived from CHO-S and expresses a bispecific antibody of the BEAT® format. It was generated by the co-transfection of five different plasmids expressing the light chain, heavy chain, single-chain Fv and two resistance genes, respectively.

The two cell lines underwent two rounds of limiting dilution that were performed in a chemically defined medium without supplementation of FCS (Fetal Calf Serum). In the limiting dilution 1 (LD1), the cells were seeded in 96 well plates at a density of 4000-10,000 cells/well, which in the presence of selection pressure (2 different antibiotics) led to <20% of wells showing growth. The resulting cell populations underwent a second limiting dilution (LD2). The cells were seeded at a density of 0.1 cells/well in 96 well plates leading to cell populations that were frozen in a first bank named the research cell bank (RCB). The master cell bank (MCB) was generated by thawing and expanding a vial of the RCB.

Following the regulatory agency guidance, a cell line development process can ensure monoclonality if at least 2 limiting dilution steps with a seeding density $\leq$ 1 cell/well have been performed prior the establishment of the cell bank. As the process followed for the two cell lines is not compliant with this guidance (LD1 seeding density > 1 cell / well), the cell population frozen in the RCB cannot be considered monoclonal. In order to obtain control populations that can be considered monoclonal, an additional round of limiting dilution (LD3) was performed on the RCB with a seeding density of 0.1 cells/well in 96 well plates.

The samples used for monoclonality demonstration of the RCB were generated by an additional LD3 (LD3$_{Serum}$) on the RCB with a seeding density of 0.1 cells/well in 96 well plates in the presence of 10% FCS. Growing wells were amplified in 96 deep well plates and cell pellets were frozen for further analysis by qPCR.

A summary of the cell populations used in this study is shown in Figure 1.

TLA/NGS

TLA followed by NGS, as well as bioinformatic analysis were performed by Cergentis (Utrecht, The Netherlands). Primer sequences can be found in Tables 1 and 2. After TLA, PCR products were sequenced on an Illumina, Illumina Miseq or Illuminal Miniseq sequencer (Illumina NexteraXT protocol for library preparation). Mapping was performed using BWA-SW (Smith Waterman alignment tool) with CriGri_1.0 (GCA_000419365.1) as reference genome.

**Table 1:** Primers used for TLA reactions for both cell lines. Each transgene is targeted by one specific primer set. One additional primer set targeting the backbone sequence common to all the transgenes was also used for TLA reactions.

| Cell lines | Transgene identity | Primer identity | Sequence |
|---|---|---|---|
| CHO-S[mAb] | LC | mAb LC A | SEQ ID NO: 1 |
| | | mAb LC B | SEQ ID NO: 2 |
| | HC | mAb HC A | SEQ ID NO: 3 |

(continued)

| Cell lines | Transgene identity | Primer identity | Sequence |
|---|---|---|---|
| | | mAb HC B | SEQ ID NO: 4 |
| | puromycine | mAb puro A | SEQ ID NO: 5 |
| | | mAb puro B | SEQ ID NO: 6 |
| | neo | mAb neo A | SEQ ID NO: 7 |
| | | mAb neo B | SEQ ID NO: 8 |
| | For all plasmids | Universal A | SEQ ID NO: 9 |
| | | Universal B | SEQ ID NO: 10 |
| CHO-S[BEAT] | LC | BEAT LC A | SEQ ID NO: 11 |
| | | BEAT LC B | SEQ ID NO: 12 |
| | HC | BEAT HC A | SEQ ID NO: 13 |
| | | BEAT HC B | SEQ ID NO: 14 |
| | scFv-Fc | BEAT scFv-Fc A | SEQ ID NO: 15 |
| | | BEAT scFv-Fc B | SEQ ID NO: 16 |
| | neo | BEAT neo A | SEQ ID NO: 17 |
| | | BEAT neo B | SEQ ID NO: 18 |
| | puro | BEAT puro A | SEQ ID NO: 19 |
| | | BEAT puro B | SEQ ID NO: 20 |
| | For all plasmids | Universal A | SEQ ID NO: 9 |
| | | Universal B | SEQ ID NO: 10 |

**Table 2:** Primers used for TLA reactions for the mixture experiment. The transgene in which a specific deletion was detected was targeted with two different primer sets.

| Transgene identity | Sequence |
|---|---|
| Set 1 A | SEQ ID NO: 21 |
| Set 1 B | SEQ ID NO: 22 |
| Set 2 A | SEQ ID NO: 23 |
| Set 2 B | SEQ ID NO: 24 |

PCR

Integration sites and plasmid-plasmid junctions identified by TLA and NGS were confirmed by standard PCR on genomic DNA, followed by Sanger sequencing (Fasteris, Plan-les-Ouates, Switzerland). Primer sequences can be found in Table 3.

**Table 3:** Primers used for PCR confirmation of the integration sites and plasmid-plasmid junctions identified by TLA/NGS ([†]binding upstream in the plasmid sequence obtained with TLA results).

| Cell line | Primer identity | Sequence |
|---|---|---|
| CHO-S[mAb] | Integration site A 5' end 1 | SEQ ID NO: 25[+] |
| | Integration site A 5' end 2 | SEQ ID NO: 26 |
| | Integration site A 3' end 1 | SEQ ID NO: 27 |

(continued)

| Cell line | Primer identity | Sequence |
|---|---|---|
|  | Integration site A 3' end 2 | SEQ ID NO: 28 |
|  | Integration B 5' end 1 | SEQ ID NO: 29 |
|  | Integration B 5' end 2 | SEQ ID NO: 30 |
|  | Integration B 3' end 1 | SEQ ID NO: 31 |
|  | Integration B 3' end 2 | SEQ ID NO: 32 |
| CHO-S[BEAT] | Junction 1 1 | SEQ ID NO: 33 |
|  | Junction 1 2 | SEQ ID NO: 34 |
|  | Junction 2 1 | SEQ ID NO: 35 |
|  | Junction 2 2 | SEQ ID NO: 36 |
|  | Junction A 1 | SEQ ID NO: 37 |
|  | Junction A 2 | SEQ ID NO: 38 |

qPCR

All qPCR assays were performed by Microsynth AG (Balgach, Switzerland). The qPCR assays for CHO-S[mAb] were performed on the LightCycler 48011 instrument in 384-well plates using SYBRGreen chemistry.

The qPCR assays for CHO-S[BEAT] were performed on the LightCycler 48011 instrument and on the Rotorgene 6000 instrument using Quantifast Multiplex Mastermix.

Primers and hydrolysis probes sequences can be found in Table 4.

**Table 4:** Primers used for qPCR analysis of sub-clones, targeting integration sites or plasmid-plasmid junctions identified by TLA/NGS.

| Cell line | Primer ID | Sequence |
|---|---|---|
| CHO-S[mAb] | WT_FW1 | SEQ ID NO: 39 |
|  | WT_REV1 | SEQ ID NO: 40 |
|  | Tg_FW1 | SEQ ID NO: 41 |
|  | Tg_REV1 | SEQ ID NO: 42 |
| CHO-S[BEAT] | WT_2_FW1 | SEQ ID NO: 43 |
|  | WT_2_REV1 | SEQ ID NO: 44 |
|  | WT2_PROBE1 | SEQ ID NO: 45 |
|  | junction_1_FW1 | SEQ ID NO: 46 |
|  | junction_1_REV1 | SEQ ID NO: 47 |
|  | junction_1_PROBE1 | SEQ ID NO: 48 |
|  | junction_2_FW1 | SEQ ID NO: 49 |
|  | junction_2_REV1 | SEQ ID NO: 50 |
|  | junction_2_PROBE1 | SEQ ID NO: 51 |
|  | junction_A_FW1 | SEQ ID NO: 52 |
|  | junction_A_REV1 | SEQ ID NO: 53 |
|  | junction_A_PROBE1 | SEQ ID NO: 54 |

**Example 3: Population drifts**

An important point to consider in the analysis for monoclonality is the impact of different growth rates in sub-populations, which can lead to important population redistributions. The specific growth rate $\mu$ for the clone CHO-S[BEAT] was determined to have a mean of 0.038 h-1 (monitored over ~80 generations). A slower growing clone ("CHO-S[BEAT-slow]") derived from the same transfection was found to have a specific growth rate of 0.033 h-1 (monitored over 36 generations). While the difference in growth is relatively modest (13%), Figure 2 shows the tremendous population reorganization over time by the overgrowth of CHO-S[BEAT] over CHO-S[BEAT-slow], assuming that one cell of each of the two clones were distributed in the same well during the limiting dilution. By the time of the RCB establishment in the CHO-S[BEAT] development campaign, the fraction of the potential contaminating clone could have been as low as 0.25 %. For the MCB (generated after additional 10 population doubling levels) this fraction would be even lower, representing less than 0.1% of the total cell population. This example shows that a cellular population should be investigated at a time point as early as possible after the cloning step, in order to minimize a population drift due to overgrowth during the cultivation phase. In this study the analysis of monoclonality was performed at the RCB level (first cell bank following the LD2 step in our process) to minimize this potential drift. This also shows that retrospective demonstration of monoclonality is analytically highly challenging, as the contaminating populations may represent only minor fractions of the total cell population.

**Example 4: Genetic signature identification by TLA/NGS**

Analysis of cell populations by TLA/NGS

TLA was identified to be the most suitable approach to analyze the integration site. The enrichment of the DNA flanking the integrated transgenes before the generation of NGS data allowed to generate a high sequence coverage specifically in the genomic region of the integrated plasmid DNA.
In this study, RCB samples from both cell lines were subjected to TLA followed by NGS in order to determine the genomic integration site(s) including plasmid - host cell DNA fusions and the architecture of the plasmids concatemer(s), i.e. plasmid - plasmid fusions and plasmid sequence integrity.
In order to confirm the unique genetic signature (and exclude unexpected site-specific integration), 47 cell populations derived from 15 different parental pools generated in several cell line development projects were analyzed using TLA and NGS in order to determine the transgene integration sites. None of them shared the same insertion site, unless they were derived from the same parental cell pool and thus descendants of the same integration event (Table 5).

| Clone families (number of analyzed samples) | | | | | | | | | | | | | | | | |
|---|---|---|---|---|---|---|---|---|---|---|---|---|---|---|---|---|
| I ( CHO-S[mAB]) | | | II (1) | III (2) | IV (1) | V (2) | VI (1) | VII (3) | VIII (1) | IX (1) | X (1) | XI (1) | XII (3) | XIII (1) | XIV (1) | XV (14) |
| Clone and 10 subclones (11) | Parental pool (1) | Clones generated from different parental pools (2) | | | | | | | | | | | | | | |
| G 1 | x | x | | | | | | | | | | | | | | |

| | | | | | | | | | | | | | | | | | |
|---|---|---|---|---|---|---|---|---|---|---|---|---|---|---|---|---|---|
| 2 | x | x | | | | | | | | | | | | | | | |
| 3 | x | x | | | | | | | | | | | | | | | |
| 4 | x | x | | | | | | | | | | | | | | | |
| 5 | | x | | | | | | | | | | | | | | | |
| 6 | | | x | | | | | | | | | | | | | | |
| 7 | | | | x | | | | | | | | | | | | | |
| 8 | | | x | | | | | | | | | | | | | | |
| 9 | | | | | x | | | | | | | | | | | | |
| 10 | | | | | | x | | | | | | | | | | | |
| 11 | | | | | | | x | | | | | | | | | | |
| 12 | | | | | | | | x | | | | | | | | | |
| 13 | | | | | | | | | x | | | | | | | | |
| 14 | | | | | | | | | | x | | | | | | | |
| 15 | | | | | | | | | | | x | | | | | | |
| 16 | | | | | | | | | | | x | | | | | | |
| 17 | | | | | | | | | | | | x | | | | | |
| 18 | | | | | | | | | | | | x | | | | | |
| 19 | | | | | | | | | | | | x | | | | | |
| 20 | | | | | | | | | | | | x | | | | | |
| 21 | | | | | | | | | | | | x | | | | | |
| 22 | | | | x | | | | | | | | | | | | | |
| 23 | | | | | | | | | | | | | x | | | | |
| 24 | | | | | | | | | | | | | x | | | | |
| 25 | | | | | | | | | | | | | x | | | | |
| 26 | | | | | | | | | | | | | x | | | | |
| 27 | | | | | | | | | | | | | x | | | | |
| 28 | | | | | | | | | | | | | x | | | | |
| 29 | | | | | | | | | | | | | | x | | | |
| 30 | | | | | | | | | | | | | | x | | | |
| 31 | | | | | | | | | | | | | | x | | | |
| 32 | | | | | | | | | | | | | | | x | | |
| 33 | | | | | | | | | | | | | | | x | | |
| non identified | | | | | | | | | | | | | | | | x | x |

Table 5: Analysis of genomic integration sites in 47 cell populations generated in several cell line development campaigns. No cell populations shared the same integration sites except when they were derived from the same parental pool ("clone family").

For the CHO-S[mAb] cell line, plasmid concatemers were integrated into the host cell genome at 4 different sites (Table 5). The same integration sites were found in the parental pool of this clone, as well as in 10 control sub-clones of this clone obtained after LD3. The parental pool contained several additional integration sites, revealing a genetic heterogeneity of the cell population at the pool level. The presence of exactly the same integration sites in the 10 control sub-clones demonstrated a certain level of homogeneity of the cell population in the RCB.

For the CHO-S[BEAT] cell line, however, no specific transgene integration site could be detected by TLA/NGS, potentially due to the absence of a matching sequence for the insertion point in the CHO reference genome.

In addition to genomic integration sites, plasmid-plasmid junction sequences in the plasmids concatemer(s) could be determined using the TLA/NGS data. Three plasmid-plasmid junctions were of special interest in the cell line CHO-S[BEAT] as they represented unique combinations of DNA sequences resulting from DNA repair. Junction 1 and Junction 2 contained stretches of non-plasmid related nucleotides and Junction A was the result of a recombination event between two plasmid parts distant from the linearization site (Figure 3). These three plasmid-plasmid junctions were detected in the TLA/NGS datasets for CHO-S[BEAT] clone, as well as in ten sub-clones generated from this clone and in the parental pool, but not in 29 unrelated cellular populations generated in the same transfection.

In the case of the CHO-S[mAb] cell line, a specific deletion in the plasmid backbone was detected: a stretch of the plasmid backbone lacked completely coverage of NGS data, and fusion reads of the sequence flanking the deletion were detected.. This deletion was detected in the clone as well as in the control sub-clones, but not in clones derived from other parental pools. Therefore, this deletion was considered a specific signature of the clone. In the parental pool of the clone of interest, reads were found in the deletion but the overall coverage was lower than for the rest of the plasmid, showing once more the heterogeneity of the parental pool, but also that the detection of reads spanning the deletion might be used as a marker for the presence of a contaminant cell line.

### Direct monoclonality assessment using TLA/NGS

The deletion in the parental pool of the CHO-S[mAb] cell line was used for the direct assessment of cell population homogeneity using TLA/NGS (Figure 4A). To determine the limit of detection of a contaminating population, a mixing experiment was performed. Increasing amounts (0.01-10%) of a contaminant cell line (derived from the same transfection, but without the specific backbone deletion) were mixed with a monoclonal sub-clone of CHO-S[mAb] (generated by the LD3). The mixed samples were then subjected to TLA/NGS with two primer pairs. A significant amount of reads could be detected in the region deleted in CHO-S[mAb] when the contaminant population fraction was >1% (Figure 4B). However, this direct approach is limited to cell lines that have an identified specific genetic feature with good coverage in TLA/NGS.

### Confirmation of the sequences by standard PCR and Sanger sequencing

Both genomic integration sites and plasmid-plasmid junctions are defined by precise DNA sequences (two fusions sequences between host cell genomic DNA and plasmid DNA - one at each end of the plasmid concatemer - for genomic integration site, and one fusion sequence for plasmid-plasmid junctions). In order to confirm the integration sites and plasmid-plasmid junctions identified by NGS in the two cell lines, genomic DNA was isolated and standard PCR were performed, targeting the fusions sequences. Size (agarose gels) and sequence (Sanger sequencing) of the resulting PCR products were confirmed which demonstrated that the sequences identified by NGS were indeed present in the cell samples (data not shown).

### Example 5: Monoclonality assessment by qPCR and statistical analysis

### Subcloning and qPCR analysis

The most suitable way to analyze the composition of a population of cells is to generate sub-clones and analyze the resulting clonal populations. The sub-cloning procedure was chosen in order to assure regulatory compliant monoclonality of the resulting population. The limiting dilution LD3$_{Serum}$ was performed in the presence of FCS in order to ensure that the resulting clones were representative of the starting population. The median value of the resulting survival rates was considered sufficiently high to make a preferential survival of specific sub-populations highly unlikely (Figure 5). A total of 458 clones for CHO-S[mAb] and 767 clones CHO-S[BEAT] were generated.

While TLA/NGS is not suited for high-throughput analysis, the genomic insertion site and plasmid-plasmid junctions can be detected and quantified using qPCR which allows high-throughput analysis.

For the plasmid-plasmid junctions, qPCR assays (J1, J2 and JA) were designed with a hydrolysis probe spanning the actual junction site between the two plasmid sequences to ensure enough specificity for the assays. The reference DNA sequence (WT assay) for CHO-S[BEAT] was a randomly chosen suitable location in the genome (Figure 6A).

As genomic insertion sites allow more specific primer design than plasmid-plasmid junctions, qPCR based on SYBR green incorporation was found sufficiently specific for CHO-S[mAb]. One of the primers was designed to bind the genomic DNA sequence on one side of the integration site B. Two other primers were designed to bind on the integrated DNA (for the transgene assay, Tg) and on the genomic DNA on the other side of the integration site (for the reference gene assay, WT), respectively. The first reaction would result in product amplification only if the transgene was present. The WT assay resulted in product amplification in the presence or absence of the transgene, as the cells contain at least 2 copies of each chromosome (see Figure 6B). In both cases, the normalization of the transgene signal (Tg or Junction) with the reference signal (WT) allowed the estimation of the abundance of the transgene in the cell genome and the detection of a potential contaminant cell lines.

The qPCR assays were qualified and LOD and LOQ were determined for each assay using a mixture of the clone of interest and an unrelated clone generated during the same transfection but not bearing the specific feature (Table 6). For Junction 2, neither LOD nor LOQ could be determined because of the lack of overall efficacy of the reaction. Nevertheless, the assay was considered suitable to determine the presence or absence of the plasmid-plasmid junction in the cell samples.

Table 6: Qualification of qPCR + LOD/LOQ determination. The assay for Junction 2 was not suitable for quantification

| | Assay qualification | | | | LOD and LOQ determination | | | | | | |
|---|---|---|---|---|---|---|---|---|---|---|---|
| | Slope | R2 | Efficiency | Log | RMSE | Slope | Intercept | LOD (signal) | LOQ (signal) | LOD (% contamina nt cells) | LOQ (% contamin ant cells) |
| Integratio n site WT | -3.416 | 0.9998 | 1.97 | 5 | | | | | | | |
| Integratio n site Tg | -3.324 | 0.9997 | 2.01 | 5 | 0.484 | 4.356 | 1.679 | 3.277 | 6.521 | 26.83 | 52.64 |
| Junction 1 | -3.28 | 0.996 | 2.02 | 5 | 0.011 | 0.045 | 0.134 | 0.169 | 0.239 | 43.51 | 70.01 |
| Junction 2 | -2.975 | 0.997 | 2.17 | 3 | 18.600 | 379.272 | 29.535 | N/A | N/A | N/A | N/A |
| Junction A | -2.496 | 0.9998 | 1.93 | 4 | 0.190 | 0.968 | 0.803 | 1.428 | 2.699 | 39.27 | 66.21 |
| Plasmid plasmid fusion WT | -3.5833 | 0.978 | 1.90 | 5 | | | | | | | |

For CHO-S[mAb], 219 clones were analyzed, amongst which 217 were positive for both WT and transgene assays. No secondary population could be detected in these clones (Figure 7). One sample (P2H07) was positive for Tg, but not for WT, suggesting that a rearrangement occurred at this locus in this particular sub-clone. The fact that the Tg assay was positive in this clone proved that it contained the plasmid integrated at the same position as the parental clone and thus that it was derived from the same progenitor. The sample P2B01 was positive for WT, but negative for Tg. The Tg assay was designed to amplify only one side of the integration site B. A standard PCR amplification followed by Sanger sequencing of the PCR product showed that the other end of the integration site was still present in this sub-clone genome. Further, the presence of the integration site A was confirmed by PCR and Sanger sequencing for this sub-clone and the RCB (data not shown). The presence of these two insertion sequences in the sub-clone genome confirmed that it was indeed derived from the same progenitor as the other sub-clones.

For CHO-S[BEAT], 207 sub-clones were analyzed for the three different plasmid-plasmid fusions identified by TLA/NGS. The clone P2B06 was negative for the Junction 2, and the clone P8H10 showed the presence of a secondary contaminating population (71%) having lost the Junction 1 (Figure 8A). Junction A was amplified successfully in all the sub-clones, without signs of contaminating populations (Figure 8B). Altogether, the results of the qPCR analysis showed that for CHO-S[BEAT] the 207 subclones were derived from a single parental progenitor. In two sub-clones, recombinant events had led to the loss or partial loss of one of the three clone-specific plasmid-plasmid junctions, the two remaining plasmid-plasmid junctions were still detected in these clones.

Statistical assessment of parental cell line homogeneity

The binomial ("contaminating population detected"/"no contaminating population detected") score method of confidence interval (Chapter 2.2 in (Wallis, Journal of Quantitative Linguistics, 20(3), 178-208, 2013)) was used to determine the probability that the parental cell line is monoclonal using SAS/JMP® software, see Formula 1 below.

(Formula 1)

$$\frac{p + \frac{z^2}{2(Clones)}}{1 + \frac{z^2}{(Clones)}} \pm \frac{z}{1 + \frac{z^2}{(Clones)}} \sqrt[2]{\frac{p(1-p)}{(Clones)} + \frac{z^2}{4(Clones)^2}}$$

Wherein $p$=0 indicates no contamination of the cell population and $z$=1.645, being the $z$ score for 95% confidence interval. To evaluate the population of cells that are potentially not monoclonal sample sizes from 10 to 8000 cells were evaluated, in particular for *Clones* values equal to 10, 20, 30, 60, 90, 180, 250, 500, 1000, 2000, 4000 and 8000 and the one-sided probability of monoclonal and not monoclonal are calculated using the formula derived from Wallis, 2013.

Next the following formula has been derived from the fitting of the One Sided 95% Not Monoclonal obtained from Formula 1 by the log of *Clones*(ln(*Clones*)).

(Formula 2)

$$One\ sided\ 95\%\ \text{Not monoclonal} = \text{Exp}\,((-0.0058) - 0.5029 \times ln(Clones) - 0.0824 \times ln(Clones)^2 + 0.0045 \times ln(Clones)^3)$$

This formula can be used to calculate the one-sided 95% probability of not monoclonal for a given value of *Clones*.

Table 7: Calculation of One sided 95% confidence interval for Monoclonality. The binomial ("contaminating population detected"/"no contaminating population detected") score method of confidence interval was used to determine the probability that the parental cell line is monoclonal using SAS/JMP® software. To evaluate the population of cells that are potentially not monoclonal sample sizes from 10 to 8000 cells were evaluated and the one-sided probability of monoclonal and not monoclonal were calculated.

| Clones Tested 100% Monoclonal | One Sided 95% Not Monoclonal | One Sided 95% CL Monoclonal |
|---|---|---|
| 10 | 21.31% | 78.69% |

(continued)

| Clones Tested 100% Monoclonal | One Sided 95% Not Monoclonal | One Sided 95% CL Monoclonal |
|---|---|---|
| 20 | 11.87% | 88.13% |
| 30 | 8.27% | 91.73% |
| 60 | 4.34% | 95.66% |
| 90 | 2.94% | 97.06% |
| 180 | 1.48% | 98.52% |
| 250 | 1.07% | 98.93% |
| 500 | 0.53% | 99.47% |
| 1000 | 0.27% | 99.73% |
| 2000 | 0.13% | 99.87% |
| 4000 | 0.07% | 99.93% |
| 8000 | 0.04% | 99.96% |

A generalized profiler for any sample size was developed for n of 10-8000 (Formula 2). The one-sided 95% CI was used as the risk is only one direction, "not monoclonal". For both cell lines analyzed, CHO-S[mAb] and CHO-S[BEAT], the one-sided upper 95% confidence interval for monoclonality was computed as a function of the number of sub-clones tested. (Figure 9). For CHO-S[mAb] no contaminating clone could be identified in the 219 analyzed subclones. For CHO-S[BEAT] no contaminating clone could be identified in 207 subclones. Based on these numbers, the fraction of contaminant in both clones was found to be 0% with a one-sided upper limit of the 95% CI of 1.22% for CHO-S[mAb] and 1.29% for CHO-S[BEAT], demonstrating that both RCBs are homogenous.

Conclusions

The requirement for the monoclonality of production cell lines for biological therapeutics is driven by the desire to generate a robust process ensuring product consistency and safety. A monoclonal cell line is expected to be homogeneous and thus to respond in a reproducible manner when confronted with minor process changes. Subpopulations within a polyclonal cell line may react differently when confronted with minor process changes, which may lead to measureable and unexpected differences in product quality attributes.

Monoclonality can be ensured by the cloning procedure (e.g. two rounds of limiting dilution with a seeding density ≤1 cell/well). If the cloning procedure is not sufficient to ensure the monoclonality of the cell line, a demonstration of the homogeneity of the cell line is required. However, in case of a contamination, the difference in the specific growth rate of different clones may lead to rapid overgrow of one clone over the other. Therefore the demonstration of initial monoclonality becomes more and more challenging with every generation following the cloning step, until it becomes virtually impossible. For this reason the assessment of cell bank homogeneity has to be performed as early as possible after the cloning step in cell line development. In the presented examples, the analysis was performed using the first cell bank frozen with the respective clones. It can be speculated that potentially present undetected subpopulations are likely to be further reduced or to even completely disappear in the following cell banks (e.g. MCB or WCB).

Targeted locus amplification (TLA) followed by next generation sequencing (NGS) allowed us to analyze the integration of the transgene DNA into the genome of the host cell line with a high degree of detail, including plasmid-plasmid junctions in the concatemer(s) and in some cases the genomic integration site. Some of the identified specific features (e.g. the deletion observed in clone CHO-S[mAb]) were used for a direct assessment of monoclonality using TLA/NGS, assuming that a contaminating population that survived the selection process would carry transgene DNA. This approach is further limited by the quality of the reference sequence, the relative abundance of reads spanning a certain region of the genome, the throughput and cost of TLA/NGS for qualification of the assay. For example, the TLA/NGS signal of a specific integration site may not be suitable for a direct monoclonality assessment. Even if the integration site is known, not all subpopulations may carry a detectable integration site (e.g. no integration site could be determined by CHO-S[BEAT]) and would therefore not be detected in the assay. Therefore preferably clearly defined and detectable genomic features, ideally a deletion in the genomic region (which may allow detection of all contaminating populations) or in the plasmid backbone (which may allow detection of contaminating populations carrying plasmids) should be considered for direct monoclonality determination. This direct analysis approach was shown to allow the detection of the presence of a contaminant population if the fraction is greater than 1% (due to the cost of the individual analysis of TLA/NGS, the LOD was not precisely determined). However, the contaminant population needs to carry at least one copy of the plasmid without a deletion that covers the entirety of the specific deletion in this region, otherwise it could not be detected by TLA/NGS.

A more general and cost-effective approach to determine the monoclonality of an existing cell line is to analyze sub-clones using qPCR for the presence of specific genetic features, followed by a statistical analysis. The genetic feature may be the bridging region of the transfected plasmid in the host cell genome, plasmid-plasmid fusions or any other genetic feature that is unique for a particular clone. If the assay is sufficiently sensitive, even the presence of subpopulations in the sub-clones not bearing the genetic feature may be detected.

Specific genetic features might be lost in sub-clones due to the high intrinsic plasticity of the CHO genome and therefore may no longer be detectable. In this study, 2 sub-clones for each cell line had lost one of the studied genetic features. Here, detailed analysis of additional genetic features still allowed the clear identification of the sub-clones as descendants of the same integration event. This highlights the need for the analysis of multiple genomic features in order to overcome assay limitations generated by the plasticity of the CHO genome.

Finally, the analysis of over 200 sub-clones per clone allowed us to establish the monoclonality of both cell lines. The analysis detected 0% of contaminating population with an upper limit of the 95% confidence interval of 1.22%. This means that the upper limit of the confidence interval is at 1.22% with 95% probability. This upper confidence interval is above the calculated lower level of the potentially remaining contaminant (0.2%) in the overgrowth example provided (Figure 1). An upper limit of the confidence interval below 0.2% in order to exclude a contamination at the clonality determining process step would have required the analysis of more than 1350 sub-clones (Supplementary Figure 2C). While qPCR allows high-throughput analysis, the generation, freezing and handling of more than 1350 subclones would be very labor intensive and may not be practical. The analysis of more than 200 sub-clones in this study allowed us to confirm the homogeneity of the existing cell bank with an acceptably small upper limit of the confidence interval of the monoclonality assumption of < 1.5%, while representing a reasonable experimental effort.

**Claims**

1.  A method for determining the monoclonality of a cell population comprising a plurality of cells that express a polypeptide of interest, wherein said method is **characterized by** the steps comprising:

    (a) identifying at least one cell of said cell population having at least one genetic signature;
    (b) generating at least one clone(s) of a single cell from said cell population by applying at least one single cell cloning step;
    (c) detecting which of said at least one clone(s) comprises said at least one genetic signature;
    (d) determining the absence of said at least one clone which do not comprise said at least one genetic signature;
    (e) confirming the monoclonality of said cell population by applying statistical analysis to determine within a predetermined confidence interval that the probability said cell population is not monoclonal is less than a predetermined value, wherein said predetermined value is calculated using the formula

$$\frac{p + \frac{z^2}{2(Clones)}}{1 + \frac{z^2}{(Clones)}} + \frac{z}{1 + \frac{z^2}{(Clones)}} {}^2\sqrt{\frac{p(1-p)}{(Clones)} + \frac{z^2}{4(Clones)^2}}$$

or the formula

$$One\ sided\ 95\%\ \text{Not monoclonal} = \text{Exp}\ ((-0.0058) - 0.5029 \times ln(Clones) - 0.0824 \times ln(Clones)^2 + 0.0045 \times ln(Clones)^3)$$

2.  The method of claim 1, wherein said at least one genetic signature is associated to a locus comprising the coding sequence of said polypeptide of interest or wherein said at least one genetic signature is not associated to a locus comprising the coding sequence of said polypeptide of interest.

3. The method of claims 1 and 2, wherein said predetermined value is equal to or greater than 0.05% and equal to or less than 2%.

4. The method of claims 1 to 3, wherein said predetermined confidence interval is equal to or greater than 90% and equal to or less than 98%.

5. The method of claims 1 to 4, wherein the number of said at least one clone(s) is between 5 and 10000.

6. The method of claims 1 to 5, wherein said polypeptide of interest is encoded by a coding sequence naturally present into the genome of said cell population.

7. The method of claims 1 to 6, wherein said at least one genetic signature is selected from the group comprising deletions, insertions, duplications and substitutions.

8. The method of claims 1 to 5, wherein said cell population is produced starting from a host cell which has undergone transfection of nucleic acid material comprising the coding sequence of said polypeptide of interest, and wherein said nucleic acid material integrates in the genome of said host cell.

9. The method of claim 8, wherein said at least one genetic signature results from the integration of said nucleic acid material into the host cell genome and said genetic signature is selected from the group comprising the integration sites, concatemer junctions and mutations.

10. The method of claim 9, wherein said integration site include but are not limited to the sites of insertion of said nucleic acid material in the host cell genome.

11. The method of claim 9, wherein said concatemers junction include but are not limited to the fused ends of two or more of said nucleic acid material.

12. The method claim 9, wherein said mutations include but is not limited to deletion, insertions, duplications and substitutions.

13. The method of claims 1 to 12, wherein said cell population and/or host cell is a eukaryotic cell selected from the group comprising insect, plant and mammalian cells.

14. The method of claim 13, wherein said cell population and/or host cell is a mammalian cell.

15. The method of claims 1 to 14 wherein said polypeptide of interest is an antibody or an antibody fragment.

16. A cell population subjected to the method of anyone of claims 1 to 15.

FIG. 1

EP 3 822 366 A1

30

**FIG. 2**

FIG. 3A

5' TCGTTCTTGGCCGTGGCCAACTCCAGCTGTGGTGGGGCAGGCAGGGCCATCGGGGTTAACAGGTGGCGTTCACAGCGCCT

S1

CTGTTGCCCCCGCCAGGAGGCCAACACGCCAAGAGCAGTGGCTGGGCCCCCGAAAGGCCGGGCACTGTGGCTCACGCCTG

S1          insertion          S2

TAATCCCAGCACTTTGCGATACCAAGGCGGGTGGATAACCTGAGGTAGGGAGTTCGAGACCAGCCTGACCAACATGGAGA

S2

AACCCCATCTCTACTA   3'

S2

AseI    SnaBI    SnaBI    EcoRV

2000'        4000'        6000'        8000'

Promoter    ORF    3' flanking region    ori    5' flanking region

Poly(A)    bla gene
bla promoter

J1 S1    J1 S2

EP 3 822 366 A1

FIG. 3B

EP 3 822 366 A1

## FIG. 3C

5' TGGGCAGGGCTCCGCATGGTGGGCTAGCACCGGTCGATCGAACCGAGCTGCAGTTGGACGTGGGAGTGGACACCTGTGGA

S1

GAGAAAGGCAAAGTGGATGTCATTGTCACTCAAGTGTATGGCCAGATCTCAATGGGTTTGGAGAGAGTTCAGTCCCTGCC

S1       S2

GCTCCCTCGCCATCCCGCGGCCCCGCCCTCGCTTCCGGTACTTCTCATGGGCTGCCAGAGGGTGAGGCAGCAGGAGGAGG

S2

AGTGCCGTCCGTCTTCAGGATTC    3'

S2

# FIG. 4A

**FIG. 4B**

FIG. 5A

**Quantiles**

| 100.0% | maximum | 114.58333333 |
|---|---|---|
| 99.5% | | 114.58333333 |
| 97.5% | | 104.16666667 |
| 90.0% | | 62.5 |
| 75.0% | quartile | 52.083333333 |
| 50.0% | median | 36.458333333 |
| 25.0% | quartile | 20.833333333 |
| 10.0% | | 10.416666667 |
| 2.5% | | 0.2604166667 |
| 0.5% | | 0 |
| 0.0% | minimum | 0 |

**Summary Statistics**

| Mean | 40.277778 |
|---|---|
| Std Dev | 22.594232 |
| Std Err Mean | 2.0625618 |
| Upper 95% Mean | 44.361856 |
| Lower 95% Mean | 36.193699 |
| N | 120 |
| Sum | 4833.3333 |
| Variance | 510.49934 |
| CV | 56.096025 |
| N Missing | 0 |

# FIG. 5B

EP 3 822 366 A1

## Quantiles

| | | |
|---|---|---|
| 100.0% | maximum | 114.58333333 |
| 99.5% | | 114.58333333 |
| 97.5% | | 114.58333333 |
| 90.0% | | 104.16666667 |
| 75.0% | quartile | 83.333333333 |
| 50.0% | median | 62.5 |
| 25.0% | quartile | 44.270833333 |
| 10.0% | | 31.25 |
| 2.5% | | 10.677083333 |
| 0.5% | | 0 |
| 0.0% | minimum | 0 |

## Summary Statistics

| | |
|---|---|
| Mean | 65.711806 |
| Std Dev | 26.178591 |
| Std Err Mean | 2.3897674 |
| Upper 95% Mean | 70.443784 |
| Lower 95% Mean | 60.979827 |
| N | 120 |
| Sum | 7885.4167 |
| Variance | 685.31862 |
| CV | 39.83849 |
| N Missing | 0 |

**FIG. 6B**

# FIG.7

## FIG. 8A

**FIG. 8B**

# FIG. 9A

Prediction Profiler

**FIG. 9B**

# FIG. 9C

Prediction Profiler

Europäisches
Patentamt

European
Patent Office

Office européen
des brevets

# EUROPEAN SEARCH REPORT

Application Number

EP 19 20 8964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AEBISCHER-GUMY CHRISTEL ET AL: "Analytical assessment of clonal derivation of eukaryotic/CHO cell populations", JOURNAL OF BIOTECHNOLOGY, ELSEVIER, AMSTERDAM, NL, vol. 286, 30 August 2018 (2018-08-30), pages 17-26, XP085519730, ISSN: 0168-1656, DOI: 10.1016/J.JBIOTEC.2018.08.020 * the whole document, in particular paragraph bridging left-hand and right-column on p. 24 * | 1-16 | INV. C12Q1/6881 |
| X,D | SEAN WALLIS: "Binomial Confidence Intervals and Contingency Tests: Mathematical Fundamentals and the Evaluation of Alternative Methods", JOURNAL OF QUANTITATIVE LINGUISTICS, vol. 20, no. 3, 4 July 2013 (2013-07-04), pages 178-208, XP055612284, ISSN: 0929-6174, DOI: 10.1080/09296174.2013.799918 * the whole document * | 1 | |
| X | LIETZKE R ET AL: "A statistical approach to determine monoclonality after limiting cell plating of a hybridoma clone", JOURNAL OF IMMUNOLOGICAL METHODS, ELSEVIER SCIENCE PUBLISHERS B.V.,AMSTERDAM, NL, vol. 76, no. 2, 11 February 1985 (1985-02-11), pages 223-228, XP023992576, ISSN: 0022-1759, DOI: 10.1016/0022-1759(85)90298-4 [retrieved on 1985-02-11] | 16 | |
| A | * the whole document * | 1-15 | |

TECHNICAL FIELDS SEARCHED (IPC)

C12Q

-/--

The present search report has been drawn up for all claims

| Place of search | Date of completion of the search | Examiner |
|---|---|---|
| Munich | 2 November 2020 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 20 8964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/093400 A1 (ARES TRADING SA [CH]) 8 June 2017 (2017-06-08) | 16 | |
| A | * the whole document, in particular the claims * | 1-15 | |
| X,D | KRISTA EVANS ET AL: "Assurance of monoclonality in one round of cloning through cell sorting for single cell deposition coupled with high resolution cell imaging", BIOTECHNOLOGY PROGRESS, vol. 31, no. 5, 1 September 2015 (2015-09-01), pages 1172-1178, XP055612527, ISSN: 8756-7938, DOI: 10.1002/btpr.2145 | 16 | |
| A | * the whole document * | 1-15 | |
| X | WO 2012/014208 A2 (YEDA RES & DEV [IL]; SHAPIRO EHUD Y [IL]; BEN-YEHEZKEL TUVAL [IL]) 2 February 2012 (2012-02-02) | 16 | |
| A | * the whole document, in particular claims 9-24 * | 1-15 | TECHNICAL FIELDS SEARCHED (IPC) |
| X | KERENSA J. KLOTTRUP ET AL: "Measuring the aggregation of CHO cells prior to single cell cloning allows a more accurate determination of the probability of clonality", BIOTECHNOLOGY PROGRESS, vol. 34, no. 3, 1 May 2018 (2018-05-01), pages 593-601, XP055612658, ISSN: 8756-7938, DOI: 10.1002/btpr.2500 | 16 | |
| A | * the whole document * | 1-15 | |

The present search report has been drawn up for all claims

| Place of search | Date of completion of the search | Examiner |
|---|---|---|
| Munich | 2 November 2020 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 19 20 8964

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2020

| Patent document cited in search report | | Publication date | Patent family member(s) | | Publication date |
|---|---|---|---|---|---|
| WO 2017093400 | A1 | 08-06-2017 | AU | 2016363746 A1 | 31-05-2018 |
| | | | CA | 3005386 A1 | 08-06-2017 |
| | | | CN | 108368549 A | 03-08-2018 |
| | | | EP | 3384045 A1 | 10-10-2018 |
| | | | HK | 1253370 A1 | 14-06-2019 |
| | | | JP | 2018537097 A | 20-12-2018 |
| | | | KR | 20180088867 A | 07-08-2018 |
| | | | US | 2018327830 A1 | 15-11-2018 |
| | | | WO | 2017093400 A1 | 08-06-2017 |
| WO 2012014208 | A2 | 02-02-2012 | US | 2013130311 A1 | 23-05-2013 |
| | | | WO | 2012014208 A2 | 02-02-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9209965 W

- WO 9413804 A

**Non-patent literature cited in the description**

- **COLLER ; COLLER.** *Hybridoma,* 1983, vol. 2 (1), 91-96
- **EVANS et al.** *Biotechnol Prog,* 2015, vol. 31 (5), 1172-1178
- **LEFKOVITS ; WALDMANN.** *Immunol Today,* 1984, vol. 5 (9), 265-268
- **QUIROZ ; TSAO.** *Biotechnol Prog,* 2016, vol. 32 (4), 1061-1068
- **WALDMANN ; LEFKOVITS.** *Immunol Today,* 1984, vol. 5 (10), 295-298
- **STASZEWSKI.** *Statistics in medicine,* 1990, vol. 9, 457-461
- **ZHOU et al.** *Biotechnol Prog,* 2017
- **WALSH.** *Nature Biotechnology,* 2014, vol. 32 (10), 992-1000

- **ZHU.** *Biotechnol Adv,* 2012, vol. 30 (5), 1158-1170
- **WARD ES et al.** *Nature,* 1989, vol. 341, 544-546
- **BIRD RE et al.** *Science,* 1988, vol. 242, 423-426
- **HUSTON JS et al.** *Proc. Natl. Acad. Sci. USA,* 1988, vol. 85, 5879-83
- **TOMLINSON I ; HOLLINGER P.** *Methods Enzymol.,* 2000, vol. 326, 461-79
- **HOLLIGER P et al.** *Proc. Natl. Acad. Sci. USA,* 1993, vol. 90, 6444-48
- **COLOMA MJ ; MORRISON SL.** *Nature Biotechnology,* 1997, vol. 15 (2), 159-163
- **WALLIS.** Journal of Quantitative Linguistics. 2013, vol. 20, 178-208